# EUROPEAN PATENT SPECIFICATION

(11) **EP 1 668 154 B1**
(45) Date of publication and mention of the grant of the patent: **26.11.2008**
(21) Application number: 04770537.1
(22) Date of filing: 20.09.2004
(51) Int. Cl.: C12Q 1/68

(54) **METHODS USEFUL FOR DETECTING AN ALTERATION IN A LOCUS COPY NUMBER**
ZUM NACHWEIS EINER ÄNDERUNG EINER LOCUS-KOPIENZAHL GEEIGNETE VERFAHREN
PROCEDES UTILISES POUR DETECTER UNE MODIFICATION DANS LE NOMBRE DE COPIES D'UN LOCUS

(30) Priority: 22.09.2003 US 504211 P
(43) Date of publication of application: 14.06.2006
(60) Divisional application: 08162118.7
(73) Proprietor: Trisogen Biotechnology Limited Partnership, 49 316 Petah-Tikva (IL)
(72) Inventor: HALLE, David, 90 435 Efrat (IL)
(74) Representative: Machtalère, Georges
(86) International application number: PCT/IL2004/000866
(87) International publication number: WO 2005/028674

(56) References cited:
- WO-A-99/01580
- WO-A-03/020974
- PENA S D J ET AL: "Fetal diagnosis of monosomy X (Turner syndrome) with methylation-specific PCR [5]" PRENATAL DIAGNOSIS, vol. 23, no. 9, 1 September 2003 (2003-09-01), pages 769-770, XP008041340 ISSN: 0197-3851
- MOORE MATHEW W ET AL: "A multiplex methylation PCR assay for identification of uniparental disomy of chromosome 7." HUMAN MUTATION, vol. 21, no. 6, June 2003 (2003-06), pages 645-648, XP008041204 ISSN: 1098-1004
- KUROMITSU JUNRO ET AL: "A unique downregulation of h2-calponin gene expression in Down syndrome: A possible attenuation mechanism for fetal survival by methylation at the CpG island in the trisomic chromosome 21" MOLECULAR AND CELLULAR BIOLOGY, vol. 17, no. 2, 1997, pages 707-712, XP002312806 ISSN: 0270-7306
- SLATER H R ET AL: "Prenatal diagnosis of Prader-Willi syndrome using PW71 methylation analysis--uniparental disomy and the significance of residual trisomy 15." PRENATAL DIAGNOSIS, vol. 17, no. 2, February 1997 (1997-02), pages 109-113, XP008041205 ISSN: 0197-3851
- SHAPIRO ET AL: "Asymmetry of methylation with FMR-1 full mutation in two 45,X/46,XX mosaic females associated with normal intellect" vol. 51, no. 4, 1994, pages 507-508,
- "European Patents Handbook R. 54" July 2004 (2004-07), pages 3-38/2, 3/38-3, 13/44-4, 13/44-5

## Description

Disease states in which the genetic component predominates over environmental factors are termed genetic disorders and typically fall into one of three categories: (i) disorders characterized by the absence, excess, or abnormal arrangement of one or more chromosomes; (ii) Mendelian or simply-inherited disorders, primarily caused by a single mutant gene and sub classified into autosomal dominant, autosomal recessive, or X-linked types; and (iii) multifactorial disorders caused by interaction of multiple genes and environmental factors.

Aneuploidias are the most common chromosomal abnormalities found in more than 50 % among abortuses [McConnell ID, Carr DH. Recent advances in the cytogenetic study of human spontaneous abortions. Obstet Gynecol. 1975 May;45(5):547-52]. Trisomies are lethal at the fetal or embryonic state, while autosomal trisomies are trisomies which allow fetal survival beyond birth.

Down's syndrome also known, as trisomy 21, is one of the most common genetic disorders which may be diagnosed prenatally. It is the cause of mental retardation and many physical and physiological anomalies in children born with the disorder. Many are born with congenital heart defects, and gastrointestinal abnormalities, which may be corrected by surgery. Physical features include flattened head in back, and slanted eyes, depressed nasal bridge, small hands and feet, excess skin at the back of neck at birth, reduced muscle tone and a simian crease in the palm of the hand [Down syndrome, (1994) National Down Syndrome Congress. Atlanta, GA: NDSC].

The prevalence of Down syndrome accounts for 9.2 cases per 10,000 live births in the U.S. Although the reasons for Down's syndrome occurrence are still poorly understood, it is well established that increased maternal age plays a factor. Thus, the risk of currying an embryo with a 21 trisomy increases exponentially for mothers over the age of 35. Due to the increased maternal age of mothers giving birth in the U.S., the prevalence of those at risk for having children diagnosed with Down syndrome in utero is much higher than before. Therefore, potentially all mothers over the age of 35 are considered high-risk for Down's and should be offered testing.

Current methods for prenatal screening for Down's syndrome are diverse and include, blood serum screening, ultrasound, invasive testing, genetic counseling, and chromosomal studies. Much research has been done to improve prenatal diagnosis of Down's syndrome, especially in the first trimester, but no test to date has been proven 100% accurate in diagnosing Down's syndrome.

The following summarizes current methods for prenatal screening and diagnosis of Down's syndrome.

### Non invasive testing

***Ultrasound imaging of fetus** -* This test is performed between the 12^{th}-18^{th} weeks of pregnancy. It looks for nucaltranslucency (i.e., increased nucal thickening or swelling), shortened length of long bones and sandal gap between first and second toe. It is appreciated though, that the sensitivity of sonography for detection of fetal trisomic conditions varies with the type of chromosome abnormality, gestational age at the time of sonography, reasons for referral, criteria for positive sonographic findings, and the quality of the sonography. As an estimate, one or more sonographic findings can be identified in 50% to 70% of fetuses with trisomy 21 (Down syndrome). Thus, the presence or absence of sonographic markers can substantially modify the risk of fetal Down syndrome and is the basis of the genetic sonogram. Because maternal biochemical and sonographic markers are largely independent, combined risk estimates results in higher detection rates than either alone.

***Maternal Serum Screening** -* Maternal serum screening is also known as the multiple marker screening tests including the triple marker test, which looks at serum α-fetoprotein (AFP, low levels of which are indicative of Down's syndrome); human chorionic gonadotropin (hCG, high levels of which are indicative of Down's syndrome); and unconjugated estriol (uE3, low levels of which are indicative of Down's). A fourth marker has recently been added inhibin A, high levels of which are indicative of a Down's syndrome diagnosis [Wald, Watt, and Hackshaw, (1999) The New England Journal of Medicine, vol. 341, no. 7. 461-469]. The triple marker test with the addition of inhibin A now makes the Quadruple marker test. These markers with the maternal age parameter can be used to diagnose Down's syndrome with a detection rate of about 70 % and a false positive rate of about 5 %. These markers can be used to diagnose Down's in the second trimester with AFP testing and ultrasound being used in the first trimester.

The quadruple test is now used with nucaltranslucent ultrasonography and testing for pregnancy associated plasma protein-A (PAPP-A). This method can increase the detection rate to 85 % with a 5 % false positive rate, thereby providing the most reliable non-invasive detection test for Down's syndrome currently available [Wald, Kennard, Hackshaw and McGuire, (1998) Health Technology Assessment, vol 2, no. 1. 1-124.]. It should be noted, however, that currently available serum markers provide statistic results, which are indefinite and oftentimes difficult to interpret.

### Invasive testing

***Amniocentesis** -* Amniocentesis is an invasive procedure in which amniotic fluid is aspirated to detect fetal anomalies in the second trimester. This test is recommended for women of increased maternal age, who are at greater risk for having a child with genetic anomalies such as Down's syndrome. Referral for amniocentesis may include unusually low or high levels of AFP. Amniocentesis is usually performed in the second trimester, but can be performed as early as the 11^{th} week of the pregnancy. A sample of amniotic fluid is taken at approximately 16 weeks of pregnancy. As only 20 % amniocytes are suitable for testing, the sample needs to be cultured to obtain enough dividing cells for metaphase analysis. Therefore results are available following 1-3 weeks, which can result in increased maternal anxiety, and consideration of second-third trimester termination. Karyotyping detects chromosomal disorders other than Down's syndrome. However, approximately 1 in 200 pregnancies result in miscarriage due to amniocentesis.

***Chorionic Villi Sampling** -* Chorionic villi sampling involves taking a sample of the chorionic membrane, which forms the placenta, and is formed by the fetus, therefore containing fetal cells. This test can be performed at the end of the first trimester (i.e., 10-12 weeks). The procedure is performed transcervically or transabdominally. Both methods are equally safe and effective. The procedure is quick (results are available in less than 24 hours) and may involve little or no pain. The sample (i.e., uncultured sample) is then analyzed under the microscope, looking specifically at chromosomal abnormalities. The advantages of CVS are early testing within the first trimester, and the decreased risk of maternal cell contamination. The disadvantages are increased risk of miscarriage, and cost. It is still important to look at maternal serum markers, although by the time AFP is looked at, it is to late to perform CVS. Positive results detect genetic disorders such as Down's at a rate of 60 to 70%. It is appreciated that 1 % of CVS show confined placental mosaicism, where the result obtained from the direct or cultured CVS is different to that of the fetus. The cultured CVS is grown from cells more closely related to fetal line than the direct CVS which is closer to the placenta. The risk of miscarriage is higher than that of amniocentesis. Furthermore the risk of amputation of legs and hands during CVS is relatively high.

***Interphase fluorescence in situ hybridization (FISH) of uncultured aminiocytes** -* A slide of amniotic fluid can be analyzed using fluorescent in situ hybridization (FISH). The test is done on uncultured interphase cells and can detect numerical chromosomal abnormalities. Results are available within 24 hours. A probe derived from chromosome 21 critical region is used to diagnose Down's syndrome. Another probe is used to test ploidity. The probe position may lead to false-negative results in the case of some translocations as two signals may be superimposed.

***Quantitative polymerase chain reaction (PCR) diagnostic** -* This procedure has been proven useful in the study of nondisjunction in Down's syndrome. Typically used are polymorphisms (GT)n repeats and Alu sequences within the 21 chromosome. [Petersen (1991) Am J Hum Genet, 48:65-71; Celi (1994); Messari (1996) Hum Genet, 97:150-155]. Thus, for example, fetal DNA from transcervical cell (TCC) samples obtained between the 7 and 9 weeks of gestation by endocervical canal flushing can be used. Trophoblast retrieval is adequate for PCR amplification of Y chromosome-specific DNA sequences and detection of paternal-specific microsatellite alleles. This method can accurately predict fetal sex: A trisomy 21 fetus was diagnosed in TCCs using fluorescent in situ hybridization (FISH) and semiquantitative PCR analysis of superoxide dismutase-1 (SOD 1). Later, quantitative fluorescent polymerase chain reaction (PCR) was demonstrated for simultaneous diagnosis of trisomies 21 and 18 together with the detection of DNA sequences derived from the X and Y chromosomes. Samples of DNA, extracted from amniotic fluid, fetal blood or tissues were amplified by quantitative fluorescent PCR to detect the polymorphic small tandem repeats (STRs) specific for two loci on each of chromosomes 21 and 18. Quantitative analysis of the amplification products allowed the diagnosis of trisomies 21 and 18, while sexing was performed simultaneously using PCR amplification of DNA sequences derived from the chromosomes X and Y. Using two sets of STR markers for the detection of chromosome 21 trisomies confirmed the usefulness of quantitative fluorescent multiplex PCR for the rapid prenatal diagnosis of selected chromosomal abnormalities [Pertl Obstet Gynecol. (2001) Sep;98(3):483-90].

In another study DNA was extracted from the surplus amniotic fluid and amplified in fluorescence-based PCR reactions, with three small-tandem-repeat markers located on chromosome 21. The products of the reactions were analyzed on a DNA sequencer to identify the presence of two or three copies of chromosome 21. Using this method a total of 99.6% informative results was achieved with three markers (Verma 1998). Chromosome quantification analysis by fluorescent PCR products was preformed also on non-polymorphic target genes. Rahil et al (2002) set up co-amplification of portions of DSCR1 (Down Syndrome Critical Region 1), DCC (Deleted in Colorectal Carcinoma), and RB1 (Retinoblastoma 1) allowed the molecular detection of aneuploidies for chromosomes 21, 18 and 13 respectively. Quantitative analysis was performed in a blind prospective study of 400 amniotic fluids. Follow up karyotype analysis was done on all samples and molecular results were in agreement with the cytogenetic data with no false-positive or false-negative results. Thus, diagnostic of aneuploidy by chromosome quantification using PCR on fetal DNA is a valid and reliable method. However, theses methods are very sensitive to fetal DNA purity since maternal DNA might mask the chromosome quantification.

***Detection of aneuploidy in single cells** -* This method is used in preimplantation genetic diagnosis. DNA is obtained from lysed single cells and amplified using degenerate oligonucleotide-primed PCR (DOP-PCR). The product is labeled using nick translation and hybridized together with normal reference genomic DNA. The comparative genomic hybridization (CGH) fluorescent ratio profiles is used to determine aneuploidy with cut-off thresholds of 0.75 and 1.25. Single cells known to be trisomic for chromosomes 13, 18 or 21 were analyzed using this technique [Voullaire et al (1999), Tabet (2001), Rigola et al (2001)].

The Fingerprinting system is another method of performing preimplantation genetic diagnosis. Tetranucleotide microsatellite markers with high heterozygosity, known allelic size ranges and minimal PCR stutter artifacts are selected for chromosomes X, 13, 18 and 21 and optimized in a multiplex fluorescent (FL)-PCR format (Katz et al (2002) Hum Reprod. 17(3):752-9]. However, these methods are limited for in vitro fertilization since isolating pure fraction of fetal cells from mother serum requires technical procedures which are not yet available.

PENA S. et al. ("Fetal diagnosis of monosomy X (Turner syndrome) with methylation-specific PCR" PRENATAL DIAGNOSIS, vol. 23, no. 9, 2003-09-01, pages 769-770) discloses a method of identifying an alteration in a locus copy number (monosomy X (Turner's syndrome)), the method comprising determining a methylation state of at least one gene in said locus (human X-linked FMR-1 gene), wherein a methylation state differing from a predetermined methylation state of said at least one gene is indicative of an alteration in said locus copy number (a methylation-specific PCR amplifies a 142 bp fragment from bisulphite-treated CpG island and a 84 bp fragment from the treated non-methylated promoter, both fragments being detected in normal female DNA while only the 84 but not the 142 bp fragment is detected in Turner's syndrome).

***Fetal cells in maternal circulation** -* The main advantage of this technique is that it is non-invasive and therefore the procedure itself carries no risk to the pregnancy. Can potentially be performed earlier than CVS as fetal DNA has been detected at 5 weeks.

Only a few fetal cells (trophoblasts, lymphocytes and nucleated red blood cells) are found in maternal circulation, therefore there is a need to select and enrich for these cells. Enriching techniques include flow/magnetic sorting, and double-density centrifugation. There are approximately 1-2 fetal cells/10 million maternal cells, and 50 % of the fetal cells will be unsuitable for karyotyping. Notably, lymphocytes are unsuitable for use in this technique since such cells remain in maternal circulation for a duration of few years and therefore results may be affected by former pregnancies. This method only examines a single chromosome, compared with tradition karyotyping.
a) FISH can be used to look at number of signals/cell in as many cells as possible to get proportions of cells with 3 signals. The hybridization efficiency of the probe can dramatically affect the number of signals seen (thereby skewing results).
b) Primed in situ labelling (PRINS) is based on the in situ annealing of specific and unlabelled DNA primers to complementary genomic sites and subsequent extension by PCR incorporating a labelled nucleotide.

Other methods of diagnosing Down's syndrome include coelemic fluid which is taken at 10 weeks and requires culturing and karyotyping and uterine cavity lavage/transcervical cell sampling. The latter is less invasive than amniocentesis or CVS. It is performed at 7-9 weeks and involves collection of cells lost from the placenta, thereby similar to direct CVS. However, this method subject the mother to contamination and infections.

Thus, prenatal diagnosis of chromosomal abnormalities (i.e., trisomies) in general and Down's syndrome in particular is complicated, requires outstanding technical skills, not fully effective and may lead to pregnancy loss. Due to the fact that there is no definitive prenatal testing for Down's, the risk of germinating pregnancy of a healthy fetus is high.

There is thus a widely recognized need for, and it would be highly advantageous to have, methods of detecting locus amplification, which lead to chromosomal abnormalities, which are devoid of the above limitations.

### SUMMARY

There is provided a method of identifying an alteration in a locus copy number, the method comprising determining a methylation state of at least one gene in the locus, wherein a methylation state differing from a predetermined methylation state of the at least one gene is indicative of an alteration in the locus copy number.

According to further features, the alteration in the locus copy number results from a chromosomal aberration selected from the group consisting of aneuploidy and polyploidy.

There is provided a method of identifying an alteration in a locus copy number in a subject, the method comprising:(a) obtaining chromosomal DNA of the subject; and (b) determining a methylation state of at least one gene at the locus of the chromosomal DNA, wherein a methylation state differing from a predetermined methylation state of the at least one gene is indicative of an alteration in copy number of the locus, thereby identifying the alteration in the locus copy number in the subject.

There is provided a method of prenatally identifying an alteration in a locus copy number, the method comprising: (a) obtaining fetal or embryonic chromosomal DNA including the locus; and (b) determining a methylation state of at least one gene in the fetal or embryonic chromosomal DNA including the locus, wherein a methylation state differing from a predetermined methylation state of the at least one gene is indicative of an alteration in the locus copy number thereby prenatally identifying the alteration in the locus copy number.

According to still further features in the described preferred embodiments obtaining the fetal or embryonic chromosomal DNA is effected by: (i) amniocentesis; (ii) fetal biopsy, (iii) chorionic villi sampling; and/or maternal biopsy.
According to still further features in the described preferred embodiments determining methylation state of the at least one gene is effected by: (i) restriction enzyme digestion methylation detection; (ii) bisulphate-based methylation detection; (iii) mass-spectrometry analysis; (iv) sequence analysis; and/or (v) microarray analysis.

There is provided a method of prenatally diagnosing Down's syndrome, the method comprising: (a) obtaining fetal or embryonic chromosome 21; and (b) determining methylation state of at least one gene in the fetal or embryonic chromosome 21, wherein the at least one gene is selected not amplified in Down's syndrome and whereas a state of the methylation differing from a predetermined methylation state is indicative of amplification of the fetal or embryonic chromosome 21, thereby prenatally diagnosing Down's syndrome.

According to still further features in the described preferred embodiments the at least one gene is selected from the group consisting of APP and cystathionine-β-synthase.

There is provided a method of identifying "compatible with life" genes, the method comprising: (a) determining a methylation state of a plurality of genes in amplified chromosomal sequence regions; and (b) identifying genes of the plurality of genes which exhibit a methylation state different from a predetermined methylation state, thereby identifying the "compatible with life" genes.

There is provided a method of identifying "compatible with life" genes, the method comprising: (a) determining expression level of a plurality of genes in amplified chromosomal sequence regions; and (b) identifying genes of the plurality of genes, which exhibit an expression level below a predetermined threshold, thereby identifying the "compatible with life" genes.

Determining expression level of the plurality of genes is effected at the mRNA leveL

Determining expression level of the plurality of genes is effected at the protein level.

According to still an additional aspect there is provided an article of manufacture comprising a packaging material and reagents identified for detecting alteration in a locus copy number being contained within the packaging material, wherein the reagents are capable of determining a methylation state of at least one gene in the locus and whereas a methylation state differing from a. predetermined methylation state of the at least one gene is indicative of the alteration in the locus copy number.

According to a further aspect there is provided a kit for identifying an alteration in a locus copy number, the kit comprising reagents for determining a methylation state of at least one gene in the locus, the at least one gene being selected from the group consisting of APP and cystathionine-β-synthase, wherein a methylation state differing from a predetermined methylation state of the at least one gene is indicative of the alteration in the locus copy number.

According to still further features in the described preferred embodiments the alteration in the locus copy number results from a chromosomal aberration selected from the group consisting of aneuploidy and polyploidy. The shortcomings of the presently known configurations are successfully addressed by providing methods and kits for identifying locus amplifications.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

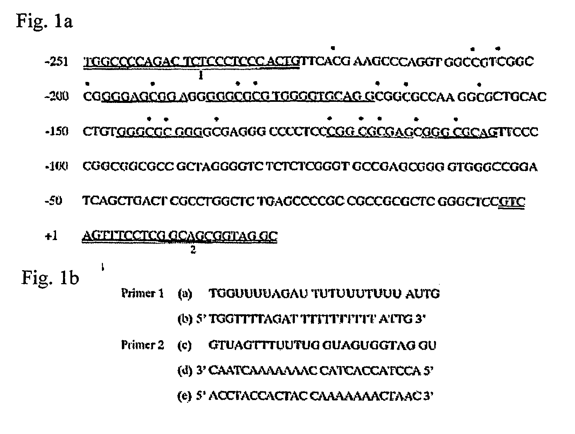

FIG. 1a is the nucleotide sequence of the amplified product of the APP promoter extending from the promoter region to the first exon of the human APP region. +1 refers to the transcription start site. Sequences used for primers 1 (SEQ ID NO: 1) and 2 (SEQ ID NO: 2) are double underlined. The six copies of the 9 bp long GC rich element are underlined. Dots above C indicate cytosine in CpG doublets in the amplified promoter region (-251 to +22).
FIG. Ibis the nucleotide sequence of the primers which were used to detect the methylation state of the DNA sequence presented in Figure 1a. Primer 1 (a-b) - designate the sequence of primer 1 (SEQ ID NO: 1, APP-F) following or prior to sulfonation, respectively; Primer 2c-e - designate the sequence of primer 2 (SEQ ID NO: 2, APP-R) following sulfonation (c), in its antisense orientation (d) or prior to sulfonation (e).

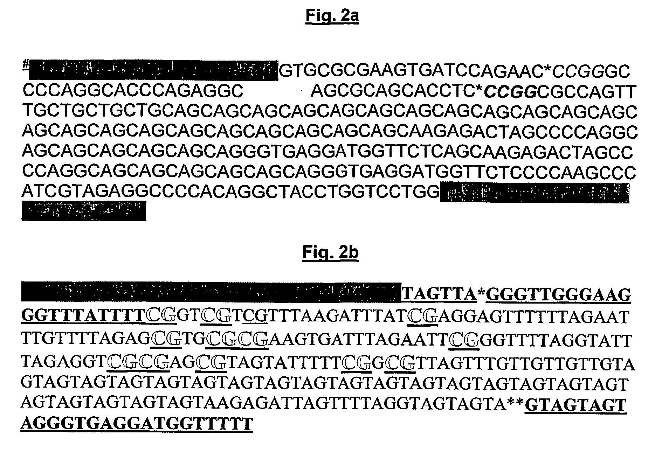

FIGs. 2a-b are the nucleotide sequences of the native (Figure 2a) and bisulfite modified (Figure 2b) sequence of Androgen receptor Exon 1. Figure 2a - # indicates the position of the forward primer; ## indicates the position of the reverse primer; * indicates a HpaII site; ** indicates a HhaI site. Figure 2b - Green highlight indicates a CpG island; Pink underline - indicates a CpG site; (#) indicates the position of AR-F-1 (SEQ ID NO: 60); (*) indicates the position of AR-F-34 primer (SEQ ID NO: 61); (**) indicates the position of AR-R-282 primer (SEQ ID NO: 62).

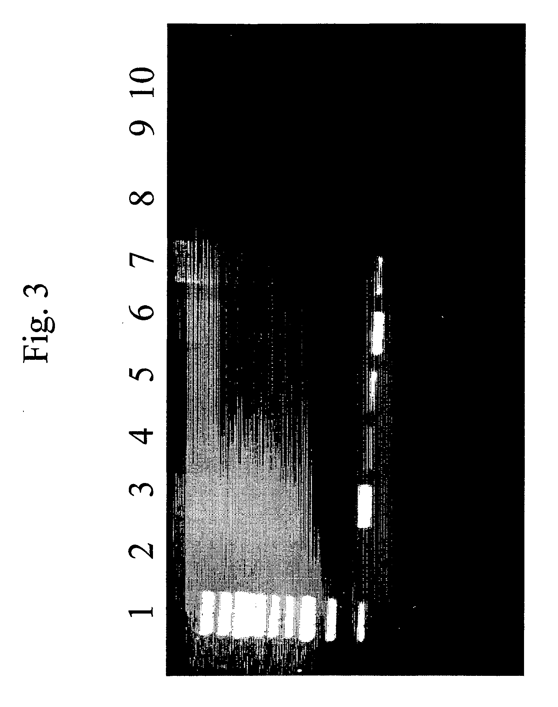

FIG. 3 is a photograph of an agarose gel visualizing the products of restriction enzyme based analysis of Androgen receptor methylation state in male, female and Kleinfelter syndrome affected subjects. Lane 1 - DNA marker; Lane 2 - negative control; Lane 3 - XX uncut; Lane 4 - XY uncut; Lane 5 - XY uncut; Lane 6 - Trisomy X uncut; Lane 7 - XX cut; Lane 8 - XY cut; Lane 9 - XY cut; Lane 10 - Trisomy X cut.

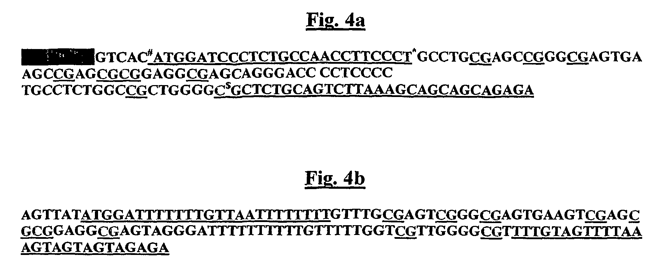

FIGs. 4a-b are the nucleotide sequences of the native (Figure 4a) and bisulfite modified (Figure 4b) DSCAM promoter. (#) - indicates position of forward primer; ($) - indicates position of reverse primer; A green highlight indicates a CpG island.

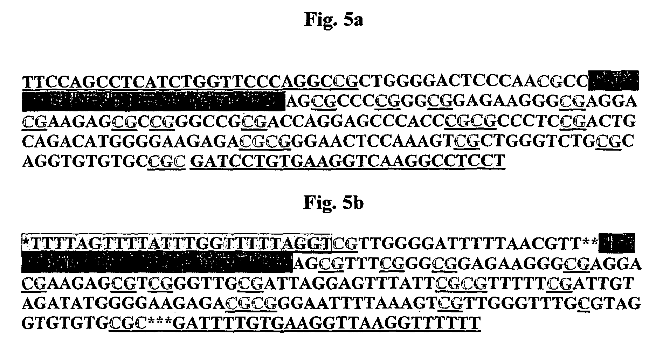

FIGs. 5a-b are the nucleotide sequences of the native (Figure 5a) and bisulfite modified (Figure 5b) IFNAR1 promoter. A green highlight indicates a CpG island. (*) indicates position of IFNR-f4-bis (SEQ ID NO: 247); (**) indicates position of IFNR-nes-f-bis (SEQ ID NO: 249); (***) indicates position of IFNR-r4 bis (SEQ ID NO: 248).

### DESCRIPTION OF THE PREFERRED EMBODIMENTS

The present invention is of methods which can be used to identify locus copy number abnormalities, which lead to chromosomal abnormalities. Specifically, the present invention can be used to prenatally detect locus amplifications such as trisomies.

The principles and operation of the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Genetic disorders are pathological conditions which are most frequently caused by variations in chromosome number such as aneuploidy, euploidy and polyploidy. Such variations in chromosome number or portions thereof are usually lethal to the embryo or fetus. Trisomies 21 (Down's syndrome), 18 (Edward's syndrome), 13 (Patau Syndrome) and sex chromosomes are the only live born autosomal trisomies. In contrast to trisomy 21, trisomies 13 and 18 disorders tend to have much more severe clinical manifestations and only rarely do affected infants survive through the first year of life. Multiple abnormalities exist in a fetus with a trisomy disorder, but there is no single anomaly that is typical for a given trisomy. Rather, there exists a characteristic constellation of clinical findings that suggests a specific diagnosis. Furthermore, since some of these patients may be mosaics for the trisomy cell line, a variety of phenotypes are possible.

To date, there is no specific treatment, therapy or cure for any trisomy disorder. For these reasons early prenatal diagnosis of chromosomal abnormalities in general and trisomies in particular is highly required.

Currently available methods for prenatal diagnosis of trisomies include sonography and cytogenetic analysis of amniocytes or chorionic cells. While sonography is limited by a high false positive rate, invasive tests are not fully effective, require high technical skills and may lead to pregnancy loss. Alternatively, diagnostic use of circulating fetal DNA in maternal plasma is currently limited to genes or mutations which are found in the fetus and not in the mother.

As is further described in the Example section which follows, while searching for a new diagnostic modality for chromosomal aberrations, the present inventors uncovered that autosomal trisomies or monosomies permit survival beyond birth, due to silencing of genes the overexpression of which is not compatible with life.

DNA methylation is a reversible mechanism by which gene expression is silenced in both prokaryotic and eukaryotic organisms. This level of control of gene expression is achieved by the ability of methyltransferases to add a methyl group to the fifth-carbon position of the cytosine pyrimidine ring especially in promoter sequence regions [Adams (1995) Bioessays 17(2):139-45]. Methylated sequences in Eukaryotic cells are usually inactive [Gold and Pedersen (1994)].

It has been clearly demonstrated that aberrant DNA methylation is a widespread phenomenon in cancer and may be among the earliest changes occurring during oncogenesis [Stirzaker (1997) Cancer Res. 57(11):2229-37]. DNA methylation has also been shown to play a central role in gene imprinting, embryonic development, X-chromosome silencing and cell-cycle regulation [Costello (2001) J. Med. Genet. 38(5):285-303]. A failure to establish a normal pattern of gene methylation is the cause for a number of genetic disorders including Rett syndrome, a major form of mental retardation, Prader-Willi syndrome, Angelman's syndrome ICF syndrome and Beckwith-Wiedmann syndrome.

In view of the central role that DNA methylation plays in gene silencing, it is highly conceivable that the same mechanism is employed to silence genes the overexpression of which is lethal (i.e., not compatible with life) suggesting that determination of a gene methylation state can be used to detect locus amplification.

In fact, while genes on chromosome 21 which are responsible for the clinical phenotype of Down's syndrome (i.e., mental retardation, congenital heart diseases and the like) are expressed at trisomic level in DS patients, there is not a significant difference in general gene expression of genes from chromosome 21 in Down's Syndrome patients as determined by microarray analysis [Gross SJ, Ferreira JC, Morrow B, Dar P, Funke B, Khabele D, Merkatz I. Gene expression profile of trisomy 21 placentas: a potential approach for designing noninvasive techniques of prenatal diagnosis. Am J Obstet Gynecol. 2002 Aug;187(2):457-62].

These findings suggest that DNA methylation acts to silence vital genes on the extra copy of chromosome 21. This assumption is further substantiated by the finding of Kuramitsu and co-workers who showed that the h2-calponin gene of chromosome 21 in Down's Syndrome patients is not overexpressed due to methylation in one of the copies of the three copies of chromosome 21 [Kuromitsu (1997) Mol. Cell Biol. 2:707-12].

This newly identified linkage between alteration in locus copy number and methylation state allows, for the first time, to effectively detect chromosomal aberrations using molecular biology techniques which are simple to execute, cost effective and pose minimal or no risk to the individual subject.

Thus, according to one aspect there is provided a method of identifying an alteration in a locus copy number.

As used herein the term "locus" refers to the position or location of a gene on a chromosome. The method according to this aspect can detect gain hereinafter, locus amplification, or loss of loci located on chromosomes 1-22, X and Y.

As used herein the phrase "locus amplification" refers to an increase in the locus copy number. Locus amplification and locus deficiency according to this aspect of the present invention may result from changes in chromosome structure (e.g., duplication, inversion, translocation, deletion insertion) and/or from an increase or decrease in chromosome number (> 2n) or portions thereof (also termed a chromosome marker). A change in chromosome number may be of an aneuploidic nature, involving a gain or a loss of one or more chromosomes but not a complete set of chromosomes (e.g., trisomy and tetrasomy). Alternatively, locus amplification may result from polyploidy, wherein three or more complete sets of chromosomes are present.

It will be appreciated that changes in chromosome number which occur only in certain cell types of the body (i.e., mosaicism) can also be detected according to this aspect of the present invention [Modi D, Berde P, Bhartiya D. Down syndrome: a study of chromosomal mosaicism. Reprod Biomed Online. 2003 Jun;6(4):499-503].

The method according to this aspect is effected by determining a methylation state (i.e., methylation pattern and/or level) of at least one gene in the locus. Methylation state which differs from a predetermined methylation state of the at least one gene is indicative of an alteration in a locus copy number.

As used herein "a predetermined state of methylation" refers to the methylation state of an identical gene which is obtained from a non-amplified locus, preferably of the same developmental state.

Thus, a change (i.e., pattern and/or increased level) in methylation state of at least one allele of the at least one gene in the above-described locus is indicative of an alteration in a locus copy number according to this aspect.

Typically, methylation of human DNA occurs on a dinucleotide sequence including an adjacent guanine and cytosine where the cytosine is located 5' of the guanine (also termed CpG dinucleotide sequences). Most cytosines within the CpG dinucleotides are methylated in the human genome, however some remain unmethylated in specific CpG dinucleotide rich genomic regions, known as CpG islands [See Antequera, F. et al., Cell 62: 503-514 (1990)]. A "CpG island" is a CpG dinucleotide rich region where CpG dinucleotides constitute at least 50% of the DNA sequence.

Therefore methylation state according to this aspect is typically determined in CpG islands preferably at promoter regions. It will be appreciated though that other sequences in the human genome are prone to DNA methylation such as CpA and CpT [see Ramsahoye (2000) Proc. Natl. Acad. Sci. USA 97:5237-5242; Salmon and Kaye (1970) Biochim. Biophys. Acta. 204:340-351; Grafstrom (1985) Nucleic Acids Res. 13:2827-2842; Nyce (1986) Nucleic Acids Res. 14:4353-4367; Woodcock (1987) Biochem. Biophys. Res. Commun. 145:888-894].

As mentioned hereinabove, the methylation state of at least one gene in the locus is determined. Example 1-4 of the Examples section lists a number of genes which can be used to determine amplification of chromosome X, 9 and 21.

Preferably the at least one gene is selected according to an expression pattern thereof Thus, methylation of genes, which locus is amplified but exhibit no change in expression, i.e., an expression pattern which is compatible with only two gene copies, is determined. Examples of such genes are listed in Table 1, below.

**Table 1**

| ***Gene Name*** | ***Chromosoe*** | ***Location*** |
|---|---|---|
| RASSF1-Ras association | 3 | 3p21.3 |
| (RalGDS/AF-6) domain family 1 | | |
| paired box 5; paired box homeotic | 9 | 9p13 |
| gene 5 (B-cell lineage specific | | |
| activator protein); B-cell lineage | | |
| specific activator protein | | |
| tissue factor pathway inhibitor 2 | 7 | 7q22 |
| ARHI, ras homolog I | 1 | 1p31 |
| FHIT fragile histidine triad gene; | 3 | 3p14.2 |
| bis(5'-adenosyl)-triphosphatase; | | |
| dinucleosidetriphosphatase; | | |
| diadenosine 5,5"'-P1,P3-triphosphate | | |
| hydrolase; AP3A hydrolase | | |
| VHL | 3 | 3p26-p25 |
| OPCML opioid-binding cell adhesion | 11 | 11q25 |
| molecule precursor; opioid-binding | | |
| protein/cell adhesion molecule-like; | | |
| opiate binding-cell adhesion molecule | | |
| CHFR checkpoint with forkhead and | 12 | 12q24.33 |
| ring finger domains | | |
| semaphorin 3B | 3 | 3p21.3 |
| MLH1 MutL protein homolog 1 | 3 | 3p21.3 |
| COX2 prostaglandin-endoperoxide | 1 | 1q25.2-q25.3 |
| synthase 2 precursor; prostaglandin | | |
| G/H synthase and cyclooxygenase | | |
| MGMT O-6-methylguanine-DNA | 10 | 10q26 |
| methyltransferase | | |
| retinoic acid receptor beta | 3 | 3p24.1 |
| PTEN | 10 | 10q23.3 |
| phosphatase and tensin homolog; | | |
| mutated in multiple advanced cancers | | |
| 1 | | |
| RASSFIA | 3 | 3p21.3 |
| APC adenomatosis polyposis coli | 5 | 5q21-q22 |
| P15-CDKN2B | 9 | 9p21 |
| BLu protein | 3 | |
| CDHI cadherin 1, type 1, E-cadherin | 16 | 16q22.1 |
| (epithelial) | | |
| TIMP-3 tissue inhibitor of | 22 | 22q12.3 |
| metalloproteinase-3 | | |
| GSN-gelsolin | 9 | 9q33 |
| p14- p14ARF- cyclin-dependent | 9 | 9p21 |
| kinase inhibitor 2A | | |
| CDKN1C- cyclin-dependent kinase | 11 | 11p15.5 |
| inhibitor 1C | | |
| LOT1-pleiomorphic adenoma gene- | 6 | 6q24-25, |
| like 1 | | |
| PIK3CG-phosphoinositide-3-kinase, | 7 | 7q22.2 |
| catalytic, gamma polypeptide | | |
| TSLC1- immunoglobulin superfamily, | 11 | 11q23.2 |
| member 4 | | |
| RB1-Retinoblastoma 1 | 13 | 13q14.2 |
| Chfr- checkpoint with forkhead and | 12 | 12q24.33 |
| ring finger domains | | |
| HTERT- telomerase reverse | 5 | 5p15.33 |
| transcriptase | | |
| MYO18B- myosin XVIIIB | 22 | 22q12.1 |
| CASP8-Caspase-8 | 2 | 2q33-q34 |
| hSNF5/INI1-SWI/SNF related, matrix | 22 | 22q11.23 |
| associated, actin dependent regulator | | |
| of chromatin, subfamily b, member 1; | | |
| sucrose nonfermenting, yeast, | | |
| homolog-like 1; integrase interactor 1; | | |
| SWI/SNF related, matrix associated, | | |
| actin dependent regulator of | | |
| HIC1-hypermethylated in cancer) | 17 | 17p13.3 |

Methods of determining gene expression are well known in the art. Examples include but are not limited to RNA-based approaches including hybridization-based techniques using oligonucleotides (e.g., Northern blotting, PCR, RT-PCR, RNase protection, in-situ hybridization, primer extension, microarray analysis and dot blot analysis) or protein-based approached such as chromatography, electrophoresis, immunodetection assays such as ELISA and western blot analysis, immunohistochemistry and the like, which may be effected using specific antibodies. For further technical details see the Laboratory reference book available at http://www.protocol-online.org/ and other references which are cited at the Examples section which follows.

A number of approaches for determining gene methylation are known in the art including restriction enzyme digestion-based methylation detection and bisulphate-based methylation detection. Several such approaches are summarized infra and in the Example 1 of the Examples section which follows (further details on techniques useful for detecting methylation are disclosed in Ahrendt (1999) J. Natl. Cancer Inst. 91:332-9; Belinsky (1998) Proc. Natl. Acad. Sci. USA 95:11891-96; Clark (1994) Nucleic Acids Res. 22:2990-7; Herman (1996) Proc. Natl. Acad. Sci. USA 93:9821-26; Xiong and Laird (1997) Nuc. Acids Res. 25:2532-2534].

### Restriction enzyme digestion methylation detection assay

This assay is based on the inability of some restriction enzymes to cut methylated DNA. Typically used are the enzyme pairs HpaII-MspI including the recognition motif CCGG, and SmaI-XmaI with a less frequent recognition motif, CCCGGG. Thus, for example, HpaII is unable to cut DNA when the internal cytosine in methylated, rendering HpaII-MspI a valuable tool for rapid methylation analysis. The method is usually performed in conjunction with a Southern blot analysis. Measures are taken to analyze a gene sequence which will not give a difficult to interpret result. Thus, a region of interest flanked with restriction sites for CG methylation insensitive enzymes (e.g., BamHI) is first selected. Such sequence is selected not to include more than 5-6 sites for HpaII. The probe(s) used for Southern blotting or PCR should be located within this region and cover it completely or partially. This method has been successfully employed by Buller and co-workers (1999) Association between nonrandom X-chromosome inactivation and BRCA1 mutation in germline DNA of patients with ovarian cancer J. Natl. Cancer Inst. 91(4):339-46.

Since digestion by methylation sensitive enzymes (e.g., HpaII) is often partial, a complementary analysis with McrBC or other enzymes which digest only methylated CpG sites is preferable [Yamada et al. Genome Research 14 247-266 2004] to detect various methylation patterns.

### Bisulphate-based methylation detection

***Genomic sequencing** -* The genomic sequencing technique [Clark et al., (1994) supra] is capable of detecting every methylated cytosine on both strands of any target sequence, using DNA isolated from fewer than 100 cells. In this method, sodium bisulphite is used to convert cytosine residues to uracil residues in single-stranded DNA, under conditions whereby 5-methylcytosine remains non-reactive. The converted DNA is amplified with specific primers and sequenced. All the cytosine residues remaining in the sequence represent previously methylated cytosines in the genome. This method utilizes defined procedures that maximize the efficiency of denaturation, bisulphite conversion and amplification, to permit methylation mapping of single genes from small amounts of genomic DNA, readily available from germ cells and early developmental stages.

***Methylation-specific PCR (MSP)** -* This is the most widely used assay for the sensitive detection of methylation. Briefly, prior to amplification, the DNA is treated with sodium bisulphite to convert all unmethylated cytosines to uracils. The bisulphite reaction effectively converts methylation information into sequence difference. The DNA is amplified using primers that match one particular methylation state of the DNA, such as that in which DNA is methylated at all CpGs. If this methylation state is present in the DNA sample, the generated PCR product can be visualized on a gel.

It will be appreciated, though, that the method specific priming requires all CpG in the primer binding sites to be co-methylated. Thus, when there is comethylation, an amplified product is observed on the gel. When one or more of the CpGs in unmethylated, there is no product. Therefore, the method does not allow discrimination between partial levels of methylation and complete lack of methylation [See U.S. Pat. No. 5,786,146; Herman et al., Proc. Natl. Acad. Sci. USA 93: 9821-9826 (1996)]. Exemplary primers for detecting methylation indicative of amplification of chromosome 21 are provided in Example 2 of the Examples section which follows.

***Real-time fluorescent MSP (MethyLight)** -* The use of real time PCR employing fluorescent probes in conjunction with MSP allows for a homogeneous reaction which is of higher throughput. If the probe does not contain CpGs, the reaction is essentially a quantitative version of MSP. However, the fluorescent probe is typically designed to anneal to a site containing one or more CpGs, and this third oligonucleotide increases the specificity of the assay for completely methylated target strands. Because the detection of the amplification occurs in real time, there is no need for a secondary electrophoresis step. Since there is no post PCR manipulation of the sample, the risk of contamination is reduced. The MethyLight probe can be of any format including but not limited to a Taqman probe or a LightCycler hybridization probe pair and if multiple reporter dyes are used, several probes can be performed simultaneously [Eads (1999) Cancer Res. 59:2302-2306; Eads (2000) Nucleic Acids Res. 28:E32; Lo (1999) Cancer Res. 59:3899-390]. The advantage of quantitative analysis by MethyLight was demonstrated with glutathione-S-transferase-P (GSTP1) methylation in prostate cancer [Jeronimo (2001) J. Natl. Cancer Inst. 93:1747-1752]. Using this method it was possible to show methylation in benign prostatic hyperplasia samples, prostatic intraexpithelial neoplasma regions and localized prostate adenocarcinoma.

***Restriction analysis of bisulphite modified DNA** -* This quantitative technique also called COBRA (Xiong et al., 1997, supra) can be used to determine DNA methylation levels at specific gene loci in small amounts of genomic DNA. Restriction enzyme digestion is used to reveal methylation-dependent sequence differences in PCR products of sodium bisulfite-treated DNA. Methylation levels in original DNA sample are represented by the relative amounts of digested and undigested PCR product in a linearly quantitative fashion across a wide spectrum of DNA methylation levels. This technique can be reliably applied to DNA obtained from microdissected paraffin-embedded tissue samples. COBRA thus combines the powerful features of ease of use, quantitative accuracy, and compatibility with paraffin sections.

***Differential methylation hybridization (DMH)*** - DMH integrates a high-density, microarray-based screening strategy to detect the presence or absence of methylated CpG dinucleotide genomic fragments [See Schena et al., Science 270: 467-470 (1995)]. Array-based techniques are used when a number (e.g., > 3) of methylation sites in a single region are to be analyzed, First, CpG dinucleotide nucleic acid fragments from a genomic library are generated, amplified and affixed on a solid support to create a CpG dinucleotide rich screening array. Amplicons are generated by digesting DNA from a sample with restriction endonucleases which digest the DNA into fragments but leaves the methylated CpG islands intact. These amplicons are used to probe the CpG dinucleotide rich fragments affixed on the screening array to identify methylation patterns in the CpG dinucleotide rich regions of the DNA sample. Unlike other methylation analysis methods such as Southern hybridization, bisulfite DNA sequencing and methylation-speficic PCR which are restricted to analyzing one gene at a time, DMH utilizes numerous CpG dinucleotide rich genomic fragments specifically designed to allow simultaneous analysis of multiple of methylation-associated genes in the genome (for further details see U.S.-A-6,605,432).

Further details and additional procedures for analyzing DNA methylation (e.,g mass-spectrometry analysis) are available in Tost J, Schatz P, Schuster M, Berlin K, Gut IG. Analysis and accurate quantification of CpG methylation by MALDI mass spectrometry. Nucleic Acids Res. 2003 May 1;31(9):e50; Novik KL, Nimmrich I, Genc B, Maier S, Piepenbrock C, Olek A, Beck S. Epigenomics: genome-wide study of methylation phenomena. Curr Issues Mol Biol. 2002 Oct;4(4):111-28. Review; Beck S, Olek A, Walter J. From genomics to epigenomics: a loftier view of life. Nat Biotechnol. 1999 Dec;17(12):1144; Fan (2002) Oncology Reports 9:181-183; http://www.methods-online.net/methods/DNAmethylation.html; Shi (2003) J. Cell Biochem. 88(1):138-43; Adoryian (2002) Nucleic Acids Res. 30(5):e21.

It will be appreciated that a number of commercially available kits may be used to detect methylation state of genes. Examples include, but are not limited to, the EZ DNA methylation kit^{™} (available from Zymo Research, 625 W Katella Ave, Orange, CA 92867, USA),

Typically, oligonucleotides for the bisulphate-based methylation detection methods described hereinabove are designed according to the technique selected.

As used herein the term "oligonucleotide" refers to a single stranded or double stranded oligomer or polymer of ribonucleic acid (RNA) or deoxyribonucleic acid (DNA) or mimetics thereof. This term includes oligonucleotides composed of naturally-occurring bases, sugars and covalent internucleoside linkages (e.g., backbone) as well as oligonucleotides having non-naturally-occurring portions which function similarly to respective naturally occurring portions (see disclosed in U.S. Pat. Nos: 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466, 677; 5,476,925; 5,519,126; 5,536,821; 5,541,306; 5,550,111; 5,563,253; 5,571,799; 5,587,361; and 5,625,050).

Thus, for example, the most critical parameter affecting the specificity of methylation-specific PCR is determined by primer design. Since modification of DNA by bisulfite destroys strand complementarity, either strand can serve as the template for subsequent PCR amplification, and the methylation pattern of each strand can then be determined. It will be appreciated, though, that amplifying a single strand (e.g., sense) is preferable in practice. Primers are designed to amplify a region that is 80-250 bp in length, which incorporates enough cytosines in the original strand to assure that unmodified DNA does not serve as a template for the primers. In addition, the number and position of cytosines within the CpG dinucleotide determines the specificity of the primers for methylated and unmethylated templates. Typically, 1-3 CpG sites are included in each primer and concentrated in the 3' region of each primer. This provides optimal specificity and minimizes false positives due to mispriming. To facilitate simultaneous analysis of each of the primers of a given gene in the same thermocycler, the length of the primers is adjusted to give nearly equal melting/annealing temperatures.

Furthermore, since MSP utilizes specific primer recognition to discriminate between methylated and unmethylated alleles, stringent annealing conditions are maintained during amplification. Essentially, annealing temperatures is selected maximal to allow annealing and subsequent amplification. Preferably, primers are designed with an annealing temperature 5-8 degrees below the calculated melting temperature. For further details see Herman and Baylin (1998) Methylation Specific PCR, in Current Protocols in Human Genetics.

Oligonucleotides designed according to the present teachings can be generated according to any oligonucleotide synthesis method known in the art such as enzymatic synthesis or solid phase synthesis. Equipment and reagents for executing solid-phase synthesis are commercially available from, for example, Applied Biosystems. Any other means for such synthesis may also be employed; the actual synthesis of the oligonucleotides is well within the capabilities of one skilled in the art and can be accomplished via established methodologies as detailed in, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988) and "Oligonucleotide Synthesis" Gait, M. J., ed. (1984) utilizing solid phase chemistry, e.g. cyanoethyl phosphoramidite followed by deprotection, desalting and purification by for example, an automated trityl-on method or HPLC.

The hereinabove-described methodology can be used to detect pathologies which are associated with alterations in locus copy number (described above).

Examples of such pathologies include but are not limited to trisomies including trisomy 1, trisomy 2, trisomy 3, trisomy 4, trisomy 5, trisomy 6, trisomy 7, trisomy 8, trisomy 9, trisomy 10, trisomy 11, trisomy 12, trisomy 13 (Patau's syndrome), trisomy 14, trisomy 15, trisomy 16, trisomy 17, trisomy 18 (Edward's syndrome), trisomy 19, trisomy 20, trisomy 21 (Down's syndrome), trisomy 22, triplo X syndrome (Kleinfelter syndrome), triplo Y syndrome, partial trisomy 6q, trisomy 9p, trisomy 11q, trisomy 14 mosaic, trisomy 22 mosaic; monosomies such as monosomy 1 and monosomy X (Turner syndrome) tetrasomies such as teterasomy 18p; triploidy such as the triploid syndrome (see the national organization for rare diseases http://www.rarediseases.org/ and chromosomal mosaicism http://www.medgen.ubc.ca/wrobinson/mosaic/contents.htm); and cancer such as chronic myelogenous leukemia.

In order to identify alterations in locus copy number in a subject, a DNA sample is obtained from the individual subject (i.e., mammal) and analyzed as described hereinabove. Preferred subjects according to this aspect of the present teaching are humans of any developmental stage (e.g., pre-implanted embryo subjects, embryo subjects, fetal subjects, neo-natal subjects and post natal subjects).

Post natal examination is typically effected to rule out the classical chromosomal syndromes and genotyping individuals with multiple congenital anomalies (MCA), parents or siblings of individuals with chromosomal abnormalities, children of individuals with balanced or structural chromosomal anomalies, couples with histories of two or more fetal losses, couples with infertility problems, individuals with ambigious genitalia, females with primary amenorrhea, individuals with mental retardation and males and females with pubertal failure.

DNA is obtained from a biological sample of the individual subject (i.e., neo-natal, post-natal). As used herein the phrase biological sample refers to a sample of tissue or fluid isolated from an individual, including but not limited to, for example, plasma, serum, spinal fluid, lymph fluid, urine the external sections of the skin, respiratory, intestinal, and genitourinary tracts, tears, saliva, milk, blood cells, tumors, organs, and also samples of in vivo cell culture constituents. Preferably used are tissue biopsies, blood or bone marrow samples.

Blood is preferably collected in sodium heparin or EDTA-coated-tubes. Newborn requires a minimum of 1-2 ml blood, child or adult requires a minimum of 3-5 ml blood. For white blood cell analysis, cells must exceed 10,000 with 10% immature cells.

Bone Marrow (0.5-2 cc bone marrow) is collected in bone marrow transport media or sodium heparin tubes

Tissue Biopsies [3mm of specimen e.g., placenta, cord, skin (typically used for testing degree of mosaicism)] is collected in sterile physiologic saline or in sterile tissue culture media.

Typically used is DNA from peripheral blood. As normal circulating lymphocytes do not divide under culture conditions, lymphocytes are obtained and subjected to external stimulating factors (i.e., mitogens) to induce cell division (i.e., mitosis). The stimulated cells can be harvested at any time following 45-96 hours of incubation.

Once the sample is obtained genomic DNA is preferably extracted such as by using a the QIAamp blood kit which is available from Qiagen (28159 Avenue Stanford Valencia CA 91355) and analyzed as described above.

Since chromosomal abnormalities are a primary reason for miscarriage and birth defects, the above-described methodology is preferably used to identify locus amplifications in unborn infants. It is well established that methylation of fetal DNA obtained from the blood of the mother can be detected using bisulfite modification, allowing the use of the epigenetic markers of the present invention in prenatal screening [see Poon et al. (2002) Clin. Chem. 48:35-41].

Methods of obtaining DNA from embryonic (i.e., the developing baby from conception to 8 weeks of development) or fetal (i.e., the developing baby from ninth weeks of development to birth) cells are well known in the art. Examples include but are not limited to maternal biopsy (e.g., cervical sampling, amniocentesis sampling, blood sampling), fetal biopsy (e.g., hepatic biopsy) and chorionic vilus sampling (see Background section and U.S. Pat. No. 6,331,395).

Isolation of fetal DNA from maternal blood is preferably used according to this aspect of the present teaching since it is a non-invasive procedure which does not pose any risk to the developing baby [see Lo (1998) Am. J. Hum. Genet. 62(4): 768-75].

Cell free fetal DNA can be collected from maternal circulation and analyzed as described above [see Bauer (2002) Am. J. Obstet. Gynecol. 186:117-20; Bauer (2001) Ann. NY Acad. Sci. 945:161-3; Pertl (2001) Obstet. Gynecol. 98:483-90; Samura (2000) Hum. Genet. 106:45-9].

Alternatively, fetal cells can be enriched from maternal blood using antibody capture techniques in which an immobilized antibody binds to fetal cells and captures the fetal cells to facilitate their enrichment [Mueller et al., "Isolation of fetal trophoblasts cells from peripheral blood of pregnant women", The Lancet 336: 197-200 (1990); Ganshirt-Ahlert et al., "Magnetic cell sorting and the transferring receptor as potential means of prenatal diagnosis from maternal blood" Am. J. Obstet. Gynecol. 166: 1350-1355 (1992)].

Fetal cells can also be labeled with antibodies and other specific binding moieties to facilitate cell sorting procedures such as flow cytometry [Herzenberg et al., "Fetal cells in the blood of pregnant women: Detection and enrichment by fluorescence-activated cell sorting", Proc. Natl Acad. Sci. (USA) 76: 1453-1455 (1979); Bianchi et al., "Isolation of fetal DNA from nucleated erythrocytes in maternal blood" Proc. Natl Acad. Sci. (USA) 87: 3279-3283 (1990); Bruch et al., "Trophoblast-Like cells sorted from peripheral maternal blood using flow cytometry: a multiparametric study involving transmission electron microscopy and fetal DNA amplification" Prenatal Diagnosis 11: 787-798 (1991). Price et al. "Prenatal diagnosis with fetal cells isolated from maternal blood by multiparameter flow cytometry" Am. J. Obstet. Gynecol 165: 1731-1737 (1991)].

PCR techniques are typically used in conjunction in order to increase the relative amount of fetal DNA and thus permit analysis [Bianchi et al., "Isolation of fetal DNA from nucleated erythrocytes in maternal blood", Proc. Natl Acad. Sci (USA) 87: 3279-3283 (1990); Adkinson et al., "Improved detection of fetal cells from maternal blood with polymerase chain reaction", Am. J. Obstet. Gynecol. 170: 952-955 (1994); Takabayashi et al., "Development of non-invasive fetal DNA diagnosis from maternal blood" Prenatal Diagnosis 15: 74-77 (1995)].

Specific configurations of prenatal diagnosis using fetal cells in the maternal circulation are disclosed in U.S. Pat. No. 6,331,395.

For example, blood (50 ml) can be obtained from a pregnant woman at 8-20 weeks gestation. The mono nuclear cell (MNC) fraction is isolated by centrifugation on Ficoll-hypaque, and cultured at 5·10⁶ / ml for 7 days in alpha medium with 10% FCS, using SCF 100 ng/ml, IL-3 100 ng/ml, and IL-6 100 u/ml. The nonadherent cells are then recovered and replated at 3·10⁵ /ml in alpha medium with 30 % FCS, 1 % BSA, 10·⁻⁴ M β-mercaptoethanol, and penicillin and streptomycin, as well as SCF 100 ng/ml, IL-3 100 ng/ml, and IL-6 100 mu/ml. All incubations are done in humidified incubators with 5% CO₂ and either room air or 5 % oxygen. After 21 days, the cells are recovered. Cells are centrifuged and DNA extracted using standard methods, for methylation analysis as described above.

Kits for enriching fetal cells from maternal blood are available from AVIVA Biosciences Corporation (San Diego, CA., http://www.avivabio.com/Technology/ fctal_cell_isolation.html).

It will be appreciated that embryonic or fetal DNA may also be obtained following fetal demise or a miscarriage. In this case, cultures are initiated from the embryonic or fetal tissue using enzymatically dissociated cells and pieces of tissue (explants). When the tissue is placed in appropriate culture conditions, the cells attach to the surface and grow as monolayers.

Chromosomal information obtained using the present methodology may be further validated using a number of cytological (e.g., Giemsa staining) and hybridization-based techniques (e.g., FISH) which are well known in the art (see for example U.S.-A-5,906,919 and 5,580,724).

Reagents for determining locus amplification as described hereinabove can be presented, in a pack or dispenser device, such as a diagnostic kit. The pack may, for example, comprise metal or plastic foil, such as a blister pack. The pack or dispenser device may be accompanied by instruction for diagnosis.

It will be appreciated that the present invention can also be used to detect pathologies which are associated with an aberrant DNA methylation mechanism which lead to abnormal methylation, as described above. Examples include but are not limited to Pradi-Willi, Angelman, Beckwith-Wiedemann, Rett and ICF syndromes. For example, the ICF syndrome is caused by abnormal function of a DNA methyltransferase enzyme termed Dnmt3b. Similarly, abnormalities in one of the proteins recognizing and binding mC (called MeCP2) cause the Rett syndrome, a form of mental retardation affecting young females.

It will be further appreciated that sex determination (e.g., prenatal) is also contemplated, since genes on the additional copy of chromosome X of females are suppressed by DNA methylation [Goto (1998) Microbiol Mol. Biol. Rev. 62(2):362-78].

There is provided a method of identifying "compatible with life" genes.

The method is effected by, determining a methylation state of a plurality of genes in amplified chromosomal sequence regions as described above.

Subsequently, genes of the plurality of genes, which exhibit a methylation state different from a predetermined methylation state are identified to thereby identify the "compatible with life" genes.

Such a method can be effectively employed to annotate genes and to identify novel therapeutic targets.

Additional objects, advantages, and features of the present invention will become apparent to one ordinarily skilled in the art upon examination of the following examples, which are not intended to be limiting. Additionally, each of the various embodiments and aspects of the present invention as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See; for example, "Molecular Cloning; A laboratory Manual" Sambrook et al., (1989); "Current Protocols in Molecular Biology" Volumes I-III Ausubel, R. M., ed. (1994); Ausubel et al., "current Protocols in Molecular Biology", John Wiley and Sons, Baltimore, Maryland (1989); Verbal "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "current Protocols in Immunology" Volumes I III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S.-A-3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. L, ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### Materials and Experimental Procedures

***DNA extraction*** - DNA is extracted from plasma and amniotic fluid samples using the QIAamp Blood kit (Qiagen, 28159 Avenue Stanford Valencia CA 91355). 800 µl of plasma or amniotic fluid is used for DNA extraction per column. DNA is eluted using 50-110 µl of elution buffer. DNA is extracted from the buffy coat of white blood using a Nucleon DNA Extraction Kit (Scotlabs Woburn, MA) according to the manufacturer's instructions.

***Bisulfite-treatment of DNA*** - DNA (up to 2 µg) is diluted in 50 µl distilled water and 5.5 µl 2M NaOH is added thereto. 5 µg of Salmon sperm DNA is then added to the reaction mixture.

The solution is incubated at 50 °C for 10 minutes to thereby generate single stranded DNA. Hydroquinone [30 µl of 10 mM hydroquinone (Sigma), freshly prepared by adding 55 mg of hydroquinone to 50 ml of water] is added to each tube. Thereafter, 520 µl of freshly prepared 3M Sodium bisulfite (Sigma S-8890, prepared by adding 1.88 gm of sodium bisulfite per 5 ml of H₂O and adjusting pH to 5.0 with NaOH] is added to the solution. Measures are taken to assure that the DNA solution is homogeneously mixed. The DNA solution is layered with mineral oil and allowed to incubate at 50°C for 16 hours or at 70 °C for 1-2 hours. Longer incubation periods are prevented to avoid methylated cytosine converting to Thymidine. Once incubation is terminated, oil is removed. 1 ml of DNA Wizard cleanup (Promega A7280) solution is added to each tube and the solution is applied to the miniprep columns in the kit. Vacuum is applied and the column is washed with 2 ml of 80% isopropanol: The DNA is eluted into clean, labeled 1.5 ml tubes by adding 50 µl warm water (i.e., 80°C). The tube is centrifuged for 1 minute and 5.5 µl of 3 M NaOH is added to each tube. The sulfonated DNA solution is incubated at room temperature for 5 minutes.

1 µl glycogen is added as carrier (Boehringer Ingelheim GmbH, Germany), 33 µl of 10 M NH₄Ac, and 3 volumes of ethanol. DNA is precipitated for at least 1 hour to overnight at -20°C and washed with 70 % ethanol. Dry pellet is resuspend in 20 µl water and stored at -20°C.

***Amplification reaction*** - 1 µl aliquot of sulfonated DNA solution is added to 50 µl of PCR reaction mixture containing 1XGC buffer 2 (TaKaRa, Shuzo, Kyoto, Japan), 2.5 mM each of dNTP, 5 U of TaKaRa LA Taqe (TaKaRa, Shuzo, Kyoto, Japan), and 50 pmol of the antisense primers. Reaction mixture is incubated at a temperature of 94 °C for 5 minutes.

Following preheating a complementary strand of the sense sequence of bisulfite-treated DNA is extended for two cycles as follows: 94°C for 1 min, 60 °C for 3 min and 72°C for 3 min.

Thereafter, 50 pmol of the sense primer is added and the mixture is heated to 94°C.

The DNAs are amplified for 8 cycles at 94°C for 1 min, 60 °C for 1.5 min and 72°C for 2 min.

Further amplification by 30 cycles at 94 °C for 1 min, 55°C for 1.5 min, and 72 °C for 2 min is effected.

Methylation in the resulting PCR product is detected by restriction enzyme analysis or direct sequencing.

***Detection of methylation site by restriction enzymes*** - The chemical modification of methylcytosine to thymine as described above change the sites of HpaII and HahI restriction enzymes such that these enzymes cannot digest the DNA in their respective sites. Cytosine methylation does not allow methylation sensitive enzymes to digest at these sites while other enzymes such as MspI which are methylation tolerant will produce a regular pattern of restriction. The use of such enzyme on bisulphate treated DNA allows to distinguish methylation sites using specific restriction enzyme (see further details in Example 3 below).

***Methylation with McrBC*** - McrBC is obtained from New England Biolabs. The enzyme is added to 5 µg of genomic DNA and reaction is incubated for overnight at 37 °C according to manufacturers instructions. The enzyme is inactivated by incubation in 65 °C for 20 minutes. 50 µg of the digested DNA is used as a template for PCR reaction. Promoter specific primers are used. Product is analyzed by agarose gel resolution.

***Direct sequencing of PCR product* -** Resultant PCR product is purified using commercially available kits (e.g., Geneclean etc.) and sequenced by commercially available automatic sequencers.

***Allele specific oligonucleotide hybridization* -** In this assay a large fragment that contain all candidate methylation sites on a gene of interest is amplified. The PCR product contain one nucleotide labeling by flurocein or other flurophore (Cy3,Cy5). The second way to label the product is by radioactive nucleotide (³²P,³³P,³⁵S, ³H or ¹⁴C) which incorpotate into the PCR product. The PCR product is than hybridized with specific oligonucleotide for methylated cytosine (i.e., thymine) vs. cytosine. The hybridization to the oligonucleotide might be done on glass or nitrocelluse using the microarray methods.

***Commercial Kits for detection mutations (or SNP)* -** The detection of methylation site can be done by commercially available "Pronto" kits of "Gamidagene" company. These kits are designed to detect mutation and/or single nucleotide polymorphisms (SNP)in conjunction with specific probes designed and configured to recognize a methylation site of interest. Similarly, other methods that can recognize a mutation in a nucleotide sequence may be used too. For example, the amplification refractory mutation system-ARMS. In this method two complementary reactions are used, one contains a primer specific for the normal allele and the other contains the mutant allele (both have a common 2nd primer). Since the PCR primer perfectly matches the variant DNA, the preferential amplification of the perfectly matched allele genotyping is identified. As describe above the methyl cytosine that is converted to thymine by bisulfite is detectable by this method.

### EXAMPLE 1

### Genes of chromosome 21

Table 2, below, lists the assigned functions of 122 genes of chromosome 21 as annotated by Gardiner and Davisson Genome Biology 2000 1(2):reviews 0002.1-0002.9. The majority have complete or presumably complete cDNA sequences. Functional annotations were assigned based on literature reports of direct experiment or on inferences from similarities to other proteins. Annotation of genes having only partial structural information was based on specific functional domain therein and are indicated by (*)(Gardiner K. http://genomebiology.com/2000/1/2/reviews/0002.1)

Functional categories were chosen to be broadly descriptive; each gene appears in only one category.

**Table 2**

| ***Functional categories*** | ***Number of*** ***genes*** | ***Functional annotations*** |
|---|---|---|
| Transcription factors, regulators, and modulators | 17 | GABPA, BACH1, RUNX1, SIM2, ERG, ETS2 (transcription |
| | | factors); ZNF294, ZNF295, Pred65, |
| | | *ZNF298, APECED (zinc fingers); KIAA0136 (leucine |
| | | zipper); GCFC (GC-rich binding protein); |
| | | SON (DNA binding domain); PKNOX1 (homeobox); |
| | | HSF2BP (heat shock transcription factor |
| | | binding protein); NRIP1 (modulator of transcriptional |
| | | activation by estrogen) |
| Chromatin structure | 4 | H2BFS (histone 2B), HMG14 (high mobility group), |
| | | CHAF1B (chromatin assembly factor), PCNT |
| | | (pericentrin, an integral component of the pericentriolar |
| | | matrix of the centrosome) |
| Proteases and protease | 6 | BACE (beta-site APP cleaving enzyme); TMPRSS2, |
| inhibitors | | TMPRSS3 (transmembrane serine proteases); |
| | | ADAMTS1, ADAMTS5 (metalloproteinases); CSTB |
| | | (protease inhibitor) |
| Ubiquitin pathway | 4 | USP25, USP16 (ubiquitin proteases); UBE2G2 (ubiquitin |
| | | conjugating enzyme); SMT3A (ubiquitin-like) |
| Interferons and | 9 | IFNAR1, IFNAR2, IL10RB, IFNGR2 (receptors/auxilliary |
| immune response | | factors); MX1, MX2 (interferon-induced); |
| | | CCT8 (T-complex subunit), TIAM1 (T-lymphoma invasion |
| | | and metastasis inducing protein), |
| | | TCP10L (T-complex protein 10 like) |
| Kinases | 8 | ENK (enterokinase); MAKV, MNB, KID2 (serine/threonine); |
| | | PHK (pyridoxal kinase), PFKL |
| | | (phosphofructokinase); ANKRD3 (ankyrin-like with kinase |
| | | domains); PRKCBP2 (protein kinase C |
| | | binding protein) |
| Phosphatases | 2 | SYNJ1 (polyphosphinositide phosphatase); PDE9A |
| | | (cyclicphosphodiesterase) |
| RNA processing | 5 | rA4 (SR protein), U2AF35 (splicing factor), RED1 (editase), |
| | | PCBP3 (poly(C)-binding protein); |
| | | RBM11 (RNA-binding motif) |
| Adhesion molecules | 4 | NCAM2 (neural cell), DSCAM; ITGB2 (lymphocyte); |
| | | c21orf43 (similar to endothelial tight junction |
| | | molecule) |
| Channels | 7 | GRIK1 (glutamate receptor, calcium channel); KCNE1, |
| | | KCNE2, KNCJ6, KCNJ15 (potassium); |
| | | CLIC11 (chloride); TRPC7 (calcium) |
| Receptors | 5 | CXADR (Coxsackie and adenovirus); Claudins 8, 14, 17 |
| | | (Claustridia); Pred12 (mannose) |
| Transporters | 2 | SLC5A3 (Na-myoinositol); ABCG1 (ATP-binding cassette) |
| Energy metabolism | 4 | ATP50 (ATP synthase oligomycin-sensitivity conferral |
| | | protein); ATP5A (ATPase-coupling factor 6); |
| | | NDUFV3 (NADH-ubiquinone oxoreductase subunit |
| | | precursor); CRYZL1 (quinone |
| | | oxidoreductase) |
| Structural | 4 | CRYA (lens protein); COL18, COL6A1, COL6A2 (collagens) |
| Methyl transferases | 3 | DNMT3L (cytosine methyl transferase), HRMTIII (protein |
| | | arginine methyl transferase); Pred28 |
| | | (AF139682) (N6-DNA methyltransferase) |
| SH3 domain | 3 | ITSN, SH3BGR, UBASH3A |
| One carbon | 4 | GART (purine biosynthesis), CBS (cystathionine-β- |
| metabolism | | synthetase), FTCD (formiminotransferase |
| | | cyclodeaminase), SLC19A1 (reduced folate carrier) |
| Oxygen metabolism | 3 | SOD1 (superoxide dismutase); CBR1, CBR3 (carbonyl |
| | | reductases) |
| Miscellaneous | 28 | HLCS (holocarboxylase synthase); LSS (lanosterol |
| | | synthetase); B3GALT5 (galactosyl transferase); |
| | | AGPAT3 (acyltransferase); STCH (microsomal stress |
| | | protein); ANABTG3 (cell cycle control); |
| | | MCM3 (DNA replication associated factor); APP |
| | | (Alzheimer's amyloid precursor); WDR4, WDR9 |
| | | (WD repeat containing proteins); TFF1,2, 3 (trefoil proteins); |
| | | UMODL1 (uromodulin); *Pred5 |
| | | (lipase); *Pred3 (keratinocyte growth factor); KIAA0653, |
| | | *IgSF5 (Ig domain); TMEM1, *Pred44 |
| | | (transmembrane domains); TRPD (tetratricopeptide repeat |
| | | containing); S100b (Ca binding); PWP2 |
| | | (periodic tryptophan protein); DSCR1 (proline rich); DSCR2 |
| | | (leucine rich); WRB (tryptophan rich |
| | | protein); Pred22 (tRNA synthetase); SCL37A1 (glycerol |
| | | phosphate permease) |

### EXAMPLE 2

### Genes of trisomy 21 and primers which can be used for detecting methylation status thereof

### Background

Deposition of fibrillar amyloid proteins intraneuronally, as neurofibrillary tangles, extracellular, as plaques and in blood vessels, is characteristic of both Alzheimer's disease (AD) and aged down's syndrome patients. The major protein found within these deposits is a small, insoluble and highly aggregating polypeptide, a4, that is thought to be derived from aberrant catabolism of its precursor, the amyloid protein precursor which is localized to chromosome 21 (21q21.2).

### Experimental Procedures

To detect amplification of the APP (GenBank Accession No. X127522), methylation of the APP promoter region is determined by bisulphite sequencing.

**Table 3**

| ***Primer ID*** | ***Oligonucleotide sequence (5'-3')*/SEQ ID** | ***Position in*** | ***PCR product*** |
|---|---|---|---|
| | **NO:** | ***X127522*** | ***size (bp)*** |
| APP-F | tggttttagattmttttttattg (1) | 3449-3473 | 272 |
| APP-R | acctaccactaccaaaaaaactaac (2) | 3696-3721 | |

Table 4 below lists preferable PCR conditions.

**Table 4**

| ***Temperture (°C)*** | ***Time*** | ***Cycle no.*** |
|---|---|---|
| 95 | 10 min | |
| 94 | 30 sec | 35 |
| 62 | 30 sec. | |
| 72 | 30 sec. | |
| 72 | 10 min | |

The resultant PCR product is sequenced to thereby identify cytosine substitution to thymidine. An amplified PCR product from the APP promoter (using primers APP-F and APP-R, Figure 1b) is shown in Figure 1a.

Alternatively, the resultant PCR product can be hybridized to an olignonucleotide microarray.

Tables 5 and 6, below, list some oligonucleotide configurations which can be used to identify methylated DNA portions on human chromosome 21 following DNA treatment with bisulfite, as described above.

**Table 5 - Amyloid precursor protein (APP) gene (GenBank Accession NoX127522) Chromosome 21**

| ***WT probe (5'-3')*/*SEQ ID NO:*** | ***Methylation probe (5'-3')*/*SEQ*** ***ID NO:*** | ***Position (gi35230)*** |
|---|---|---|
| gagggggtgtgtggg/(5) | gagggggcgtgtggg/(6) | 3509-3523 |
| gttaaggtgttgtat/(7) | gttaaggcgttgtat/(8) | 3535-3549 |
| ttgtgggtgtggggt/(9) | ttgtgggcgtggggt/(10) | 3550-3563 |
| tttttggtgtgagtg/(11) | tttttggcgtgagtg/(12) | 3573-3591 |
| gagtgggtgtagttt/(13) | gagtgggcgtagttt/(14) | 3583-3597 |
| tttggtggtgttgtta/(15) | tttggtggcgttgtta/(16) | 3598-3613 |
| ggttgttgtgtttggg/(17) | ggttgttgcgtttggg/(18) | 3677-3692 |
| tgttggttggggagt/(19) | tgttggtcggggagt/(20) | 3492-3506 |
| ttttttttggtgtga/(21) | tttttttcggtgtga/(22) | 3570-3584 |
| agttttttggtggtg/(23) | agtttttcggtggtg/(24) | 3592-3606 |
| ggtgggttggattag/(25) | ggtgggtcggattag/(26) | 3639-3653 |
| tggggagtggagggg/(27) | gggggagcggagggg/(28) | 3500-3514 |
| tttttggcgtgagtg/(29) | tttttggcgtgagtg/(30) | 3572-3586 |
| gggggtgtgtggggt/(31) | gggggtgcgtggggt/(32) | 3511-3525 |
| gtgtaggtggtgtta/(33) | gtgtaggcggtgtta/(34) | 3523-3537 |
| tttggtgtgagtggg/(35) | tttggtgcgagtggg/(36) | 3574-3588 |

**Table 6 H2-calponin gene (GenBank Accession No. gi:4758017), Chromosome 21 [Kuromitsu J, et al Mol Cell Biol. (1997) 17(2):707-12]**

| ***WT probe (5'-3')*/*SEQ ID NO:*** | ***Methylation probe (5'-3')* /** | ***Position*** |
|---|---|---|
| | ***SEQ ID NO:*** | ***(gi4758017)*** |
| aatttggtgttttta/(37) | aatttggcgttttta/(38) | 966501-966515 |
| atatttgcgttttgg/(39) | atatttgcgttttgg/(40) | 966528-966542 |
| tgtgttttgggttaa/(41) | tgtgtttcgggttaa/(42) | 966533-966547 |
| ggtgtggtgtgtgga/(43) | ggtgtggcgtgtgga/(44) | 966559-966573 |
| tgtggcgtgtggagt/(45) | tgtggcgcgtggagt/(46) | 966561-966575 |
| tggagtttggtgtgt/(47) | tggagttcggtgtgt/(48) | 966570-966584 |
| agtttggtgtgtttt/(49) | agtttggcgtgtttt/(50) | 966572-966586 |
| aattttgcgttagtt/(51) | aattttgcgttagtt/(52) | 966588-966602 |
| gttagtttggtggtt/(53) | gttagttcggtggtt/(54) | 966596-966610 |

### EXAMPLE 3

### Genes of trisomy X and primers which can be used for detecting methylation status thereof

In females one set of most genes of the duplicate X chromosome is silenced. Silencing typically occurs by CpG methylation of promoters of such genes. Several methylation analysis procedures were employed to detect the methylation status of the androgen receptor (GenBank Accession No. NM_00044) in males, females and in Kleinfelter Syndrome affected subjects.

### Experimental Procedures

***Cells*** - 12 day cultured amniocytes of male, female and Kleinfelter syndrome affected embryos were obtained from Coriell Institute NJ. Kleinfelter cells Cat. No. GMO3102. Normal cell Cat. Nos.

***DNA extraction*** - Cells were centrifuged for 10 minutes 2,500 rpm. Cell pellets were resuspended in lysis buffer including 75 mM NaCl and 25 mM EDTA and vortexed well to disintegrate plasma membrane. Thereafter, 10 % SDS solution (1/10 of the final volume) was added to the mixture and the solution was mixed by inversion. The solution was incubated over night at 55 °C in the presence of Proteinase K (10mg/ml, 1/10 of the final volume). An equal volume of Phenol:Chloroform (1:1) was added to the solution, mixed well by inversion (5 min) and centrifuged for 15 minutes at 14,000 x g to reach phase separation. Chloroform was added to the upper phase, the solution was well mixed by inversion for 5 min, centrifuged at 14,000 x g for 5 min to reach phase separation, collecting the upper phase, to which 3 M sodium acetate (1/10 of final volume) was added and mixed well by inversion. DNA was ethanol precipitated (70 %) for over night and concentration and purity were thereafter determined.

### Restriction enzyme based analysis

0.5 µg DNA molecules (i.e., bisulfite-treated or non-treated) were digested with HpaII (30 units, NEB Enzyme, New England Biolabs. Inc. Beverly MA 01915-5599 USA). To ensure complete digestion, incubation was allowed to proceed for overnight including a second addition of fresh enzyme following 8 hours of incubation.

Following digestion, 2 µl of DNA from the digestion mixture was used as template for PCR using the primers listed in Table 7 below and under the conditions described in Tables 8-9 below

**Table 7**

| **Primer** | **Sequence (5'-3')/SEQ ID NO:** | ***Position in*** | ***PCR product*** |
|---|---|---|---|
| **name** | | ***NM000044*** | ***(bp)*** |
| **AR-f** | TCCAGAATCTGTTCCAGAGCGTGC/55 | 1183-1207 | ~300* |
| **AR-r** | GCTGTGAAGGTTGCTGTTCCTCAT/56 | 1447-1470 | |

| | | | |
|---|---|---|---|
| * - the size of the PCR product depends on the number of CAG repeats in the DNA retrieved from the patient | | | |

**Table 8**

| | ***Reaction mixture*** |
|---|---|
| Buffer 10X | 0.1 of final volume (20 µl) |
| dNTPs 2mM | 0.1 of final volume (20 µl) |
| AR-f 10pmol/µl | 0.1 of final volume (20 µl) |
| AR-r 10pmol/µl | 0.1 of final volume (20 µl) |
| Water | Complete to the final volume (20 µl) |
| Enzyme* | 1 unit |
| DNA | 0.1-0.15 of final volume (20 µl) |

| | |
|---|---|
| *NEB Enzyme-Taq DNA polymerase Cat. No. M0267 New England Biokabs. Inc. Beverly MA 01915-5599 USA. | |

**Table 9**

| ***Temperature*** | ***Time*** | ***No. of cycles*** |
|---|---|---|
| 94°C | 4 min | |
| 94°C | 45 sec | 35 |
| 59°C | 45 sec | |
| 72°C | 1 min | |
| 72°C | 7 min | |

The resultant PCR product of about 300 bp was resolved and visualized on a 2.5 % agarose gel.

### Methylation specific PCR (MSP)

DNA was bisulfite treated as described in the Experimental procedures hereinabove.

Primers and PCR conditions are listed in Tables 10, 11 and 12, respectively.

**Table 10**

| **Primer** | **Sequence (5'-3')/SEQ ID NO:** | ***Position in*** | ***PCR product*** |
|---|---|---|---|
| **name** | | ***NM000044*** | ***(bp)*** |
| **AR-U** | tagaatttgttttagagtgtgtgt/57 | 1185-1208 | |
| **AR-M** | tttgttttagagcgtgcg/58 | 1189-1207 | ~225 |
| **AR-R** | aaaaccatcctcaccctact/59 | 1385-1404 | |

**Table 11**

| | ***Mix Unmethylated*** | ***Mix Methylated*** |
|---|---|---|
| Buffer 10X DS* | 0.1 of final volume | |
| dNTPs 2mM | 0.1 of final volume | 0.1 of final volume |
| AR-U 10pmol/µl | 0.1 of final volume | |
| AR-M 10pmol/µl | | 0.1 of final volume |
| AR-U 10pmol/µl | 0.1 of final volume | 0.1 of final volume |
| Water | Complete to the final volume | Complete to the final volume |
| Enzyme* | 1 unit | 1 unit |
| DNA | 0.1-0.15 of final volume | 0.1-0.15 of final volume |

| | | |
|---|---|---|
| *Buffer DS 10X - 166 mM Ammonium sulfate, 670 mM Trizma; 67mM Mg chloride; 100 mM mercaptoethanol; 1% DMSO; Ammonium sulfate-Sigma A 4418; Trizma- Sigma T 5753; DMSO-Trizma D 8414; MgC12-Sigma M-1028; Mercaptoethanol-Sigma M 3148. | | |

**Table 12**

| ***Temperature*** | ***Time*** | ***No. of Cycles*** |
|---|---|---|
| 94°C | 4 min | |
| 94°C | 45 sec | 35 |
| 59°C | 45 sec | |
| 72°C | 1 min | |
| 72°C | 7 min | |

Product identity was confirmed by a two-step nesting PCR reaction, primers of which are listed in Table 13, below and PCR conditions are listed in Tables 14-16, below. The DNA template of the first PCR was used for bisulfite modification (~50ng). PCR product was used as a template for a second PCR reaction (1/20 of final volume). Reaction product was sequenced.

**Table 13**

| **Primer** | **Sequence (5'-3')** | ***Position in*** | ***PCR product*** |
|---|---|---|---|
| **name** | | ***NM000044*** | ***(bp)*** |
| **AR-F-1** | agatttagttaagtttaaggatggaagtg/60 | 1096-1124 | |
| **AR-F-34** | gggttgggaagggtttatttt/61 | 1131-1151 | ~280* |
| **AR-R-282** | aaaaaccatcctcaccctactactac/62 | 1379-1404 | |

| | | | |
|---|---|---|---|
| *the size of the PCR product linearly correlates with the number of CAG repeats in the DNA obtained from the patient | | | |

**Table 14 - Step I**

| | ***Mix 1*** |
|---|---|
| Buffer 10X | 0.1 of final volume |
| dNTPs 2mM | 0.1 of final volume |
| AR-F-1 10pmol/µl | 0.1 of final volume |
| AR-R-282 10pmol/ µl | |
| Water | Complete to the final volume |
| DNA | 0.1-0.1 of final volume |
| Enzyme* | 1 unit |

| | |
|---|---|
| *NEB Enzyme | |

PCR conditions for amplifying exon 1 of Androgene receptor from bisulfite modified DNA are listed in Tables 15 and 16 below.

**Table 15**

| ***Temperature*** | ***Time*** | ***Cycles No.*** |
|---|---|---|
| 94°C | 4 min | |
| 94°C | 45 sec | 35 |
| 59°C | 45 sec | |
| 72°C | 1 min | |
| 72°C | 7 min | |

**Table 16 - Step II**

| | Mix 1 |
|---|---|
| Buffer 10XNEB | 0.1 of final volume |
| dNTPs 2mM | 0.1 of final volume |
| AR-F-34 10pmol/µl | 0.1 of final volume |
| AR-R-282 10pmol/ µl | 0.1 of final volume |
| Water | Complete to the final volume |
| DNA (product of step I) | 0.05 of final volume |
| Enzyme* | 1 unit |

| | |
|---|---|
| *NEB Enzyme | |

The PCR product of step II was resolved in 2.5 % agarose gel and purified by commercially available purification kit (GFX PCR cat.No, 27-9602-01 of Amersham Bioscience Piscataway Bioscience NJ 08855-USA) and then subcloned to pGEM plasmid (pGEM-T Easy Vector Vector System I Cat. No. A1360 or pGEM-T Vector Vector System I Cat. No. A3600 Promega Corporation Madison WI USA). Accurate sequencing was confirmed by sequencing of 5-10 clones of each PCR product. Sequencing was effected by an ABI Sequencer machine.

### Results

The native sequence of exon 1 of Androgen receptor along with HpaII and HhaI restriction sites is given in Figure 2a. A putative sequence obtained following bisulfite modification is shown in Figure 2b.

Figures 3 and 4 depict the results of Androgen receptor methylation state in males, females and Kleinfelter Syndrome affected subjects as determined by restriction enzyme based analysis and by methylation specific PCR (MSP).

As is shown in Figure 3, PCR amplification of HpaII treated DNA samples obtained from XY (i.e., male) subjects resulted in no product. However, the same reaction using HpaII treated DNA samples obtained from XX and XXY subjects resulted in a clear band of 280 bp, a product of Exon 1 of the Androgen Receptor exon1.

MSP analysis of the methylation state of Exon 1 of Androgen receptor from male and Kleinfelter syndrome affected subjects showed that DNA amplification using methylated primers occurred only in DNA obtained from Kleinfelter affected subjects (i.e., 46XXY).

Altogether, these results clearly support DNA methylation mediated gene silencing of the Androgen receptor in Kleinfelter Syndrome affected subjects and suggest it as a valuable diagnostic tool for this pathology.

It will be appreciated that since MSP does not efficiently detect partial methylation (i.e., not all methylation sites in a given allele are in practice methylated), the use of oligonucleotide microarray may be advantageous.

Oligonucleotides which may be efficiently used in such a microarray are listed in Table 17, below.

**Table 17**

| ***WT probe (5'-3')*/*SEQ ID NO:*** | ***Methylation probe (5'-3')* /*SEQ ID NO:*** | ***Position*** ***(M00044)*** |
|---|---|---|
| ggtttatttttggttgttgtt/63 | ggtttattttcggttgttgtt/66 | 1142-1162 |
| | ggtttatttttggtcgttgtt/67 | |
| | ggtttatttttggttgtcgtt/68 | |
| | ggtttattttcggtcgttgtt/69 | |
| | ggtttatttttggtcgtcgtt/70 | |
| | ggtttattttcggttgtcgtt/71 | |
| | ggtttattttcggtcgtcgtt/72 | |
| tatttttggttgttgtttaag/64 | tattttcggttgttgtttaag/73 | 1146-1166 |
| | tatttttggtcggtgtttaag/74 | |
| | tatttttggttgtcgtttaag/75 | |
| | tattttcggtcgttgtttaag/76 | |
| | tatttttggtcgtcgtttaag/77 | |
| | tattttcggtgtcgtttaag/78 | |
| | tattttcggtcgtcgtttaag/79 | |
| ttttggttgttgttaagattt/65 | tttcggttgttgttaagattt/80 | 1150-1170 |
| | ttttggtcgttgttaagattt/81 | |
| | ttttggttgtcgttaagattt/82 | |
| | tttcggtcgttgttaagattt/83 | |
| | ttttggtcgtcgttaagattt/84 | |
| | tttcggttgtcgttaagattt/85 | |
| | tttcggtcgtcgttaagattt/86 | |
| taagatttattgaggagtttt/89 | taagatttatcgaggagtttt/88 | 1162-1182 |
| tgttttagag *tgtgtgtg*aag/90 | tgttttagag *cgtgtgtg*aag/91 | 1192-1212 |
| | tgttttagag *tgtgcgtg*aag/92 | |
| | tgttttagag *tgtgtgcg*aag/93 | |
| | tgttttagag cgtgcgtgaag/94 | |
| | tgttttagag *cgtgtgcg*aag/95 | |
| | tgttttagag *tgtgcgcg*aag/96 | |
| | tgttttagag *cgtgcgcg*aag/97 | |
| ttagagtgtgtgtgaagtgat/98 | ttagag*cgtg**tgt*gaagtgat/99 | 1196-1216 |
| | ttagag*tgtgcgtg*aatgat/100 | |
| | ttagag*tgtgtgcg*aagtgat/101 | |
| | ttagag*cgtg**cgt*gaagtgat/102 | |
| | ttagag*cgtgtgcg*aagtgat/103 | |
| | ttagag*tgtgcgcg*aagtgat/104 | |
| | ttagag*cgtgcgcg*aagtgat/105 | |
| agagtgtgtgtgaagtgattt/106 | agag*cgtg**tgt*gaagtgattt/107 | 1198-1218 |
| | agag*tgtgcg**t*gaagtgattt/108 | |
| | agag*tgtgtgcg*aagtgattt/109 | |
| | agag*cgtgcg**t*gaagtgattt/110 | |
| | agag*cgtgtgcg*aagtgattt/111 | |
| | agag*tgtgcgcg*aagtgattt/112 | |
| | agag*cgtgcgcgc*aagtgattt/113 | |
| atttagaatttgggttttagg/114 | atttagaattcgggttttagg/115 | |
| atttagaggttgtgagtgtag/116 | atttagaggtcgcgagcgtag/117 | |
| | atttagaggtcgtgagtgtag/118 | |
| | atttagaggttgcgagtgtag/119 | |
| | atttagaggttgtgagcgtag/120 | |
| | atttagaggtcgcgagtgtag/121 | |
| | atttagaggtcgtgagcgtag/122 | |
| | atttagaggttgcgagcgtag/123 | |
| | atttagaggtcgcgagcgtag/124 | |
| atttagaggttgtgagtgtag/125 | Ttagaggtcgcgagcgtagta/126 | |
| | Ttagaggtcgtgagtgtagta/127 | |
| | Ttagaggttgcgagtgtagta/128 | |
| | Ttagaggttgtgagcgtagta/129 | |
| | Ttagaggtcgcgagtgtagta/130 | |
| | Ttagaggtcgtgagcgtagta/131 | |
| | Ttagaggttgcgagcgtagta/132 | |
| | ttagaggtcgcgagcgtagta/133 | |
| aggttgtgagtgtagtatttt/134 | Aggtcgcgagcgtagtatttt/135 | |
| | Aggtcgtgagtgtagtatttt/136 | |
| | Aggttgcgagtgtagtatttt/137 | |
| | Aggttgtgagcgtagtatttt/138 | |
| | Aggtcgcgagtgtagtatttt/139 | |
| | Aggtcgtgagcgtagtatttt/140 | |
| | Aggttgcgagcgtagtatttt/141 | |
| | aggtcgcgagcgtagtatttt/142 | |
| tagtattttttggtgttagtt/143 | tagtattttttggcgttagtt/144 | |
| | tagtatttttcggtgttagtt/145 | |
| | tagtatttttcggcgttagtt/146 | |
| tagtattttttggtgttagtttgt/147 | tagtattttttggcgttagtttgt/148 | |
| | tagtatttttcggtgttagtttgt/149 | |
| | tagtatttttcggcgttagtttgt/150 | |

### EXAMPLE 4

### Putative markers for Chromosome 21 autosomal trisomy identified according to the teachings of the present invention

As mentioned hereinabove, genes which are located on amplified chromosomes or chromosome regions are usually not overexpressed probably due to methylation of upstream promoter regions which lead to specific gene silencing.

Table 18 below, shows the ratio of chromosome 21 gene expression in amniotic cells obtained from a Down's syndrome affected subject versus amniotic cells obtained from a normal subject. A X < 1.5 ratio is indicative of gene silencing (www.hgu.mrc.ac.uk/Research/Cellgen/Supplements/Unigene/t21all.htm).

**Table 18**

| *Gene Name* | *Accession No.* | *Ratio* | *Location* | *CpGisland* | *Signe* |
|---|---|---|---|---|---|
| amyloid beta (A4) precursor protein (protease nexin-II, Alzheimer disease) | M28373 | 1.38 | 21q21.3 | Y | APP |
| ATP-binding cassette, sub-family G (WHITE), member 1 | AF038175 | 1.23 | 21q22.3 | Y | ABCG1 |
| autoimmune regulator (automimmune polyendocrinopathy candidiasis ectodermal dystrophy) | AJ009610 | 1.12 | 21q22.3 | Y | AlRE |
| BTB and CNC homology 1, basic leucine zipper transcription factor 1 | AI830904 | 1.02 | 21q22.11 | Y | BACH1 |
| BTG family, member 3 | BE896159 | 1.83 | 21q21.1-q21.2 | Y | BTG3 |
| carbonyl reductase 1 | AP000688 | 1.28 | 21q22.13 | | CBR1 |
| carbonyl reductase 3 | AB003151 | 1.06 | 21q22.2 | Y | CBR3 |
| chromatin assembly factor 1, subunit B (p60) | NM_005441 | 0.97 | 21q22.13 | Y | CHAF1B |
| | | | | | |
| chromosome 21 open reading frame | 18 AB004853 | 1.08 | 21q22.12 | Y | C21orf18 |
| chromosome 21 open reading frame 18 | AA984919 | | 21q22.12 | Y | C21orfl8 |
| chromosome 21 open reading frame 2 | AP001754 | 0.89 | 21q22.3 | Y | C21orf2 |
| collagen, type VI, alpha 1 | X99135 | 1.58 | 21q22.3 | Y | COL6A1 |
| collagen, type VI, alpha 2 | AI635289 | 1.23 | 21q22.3 | Y | COL6A2 |
| collagen, type XVIII, alpha 1 | AF018081 | 1.17 | 21q22.3 | Y | COL18A1 |
| coxsackie virus and adenovirus receptor | AI557255 | 1.23 | 21q21.1 | Y | CXADR |
| cystatin B (stefin B) | BF341232 | 1.94 | 2iq22.3 | | |
| DNA segment on chromosome 21 (unique) 2056 expressed sequence | AL137757 | 1.07 | 21q22.3 | Y | D21S2056E |
| Down syndrome cell adhesion molecule cell adhesion molecule | AF217525 | 0.9 | 21q22.2 | Y | DSCAM |
| Down syndrome critical region gene 1 | U85267 | 0.82 | 21q22.12 | Y | DSCR1 |
| Down syndrome critical region gene 3 | D87343 | 1.19 | 21q22.2 | Y | DSCR3 |
| f-box and WD-40 domain protein 1B | AA436684 | 0.9 | 21q22.11 | | |
| glutamate receptor, ionotropic, kainate 1 | NM_000830 | 0.96 | 21q21.3 | Y | GRIK1 |
| HMT1 (hnRNP methyltransferase, S. cerevisiae)-like 1 | NM_001535 | 1.15 | 21q22.3 | Y | HRMTIL1 |
| holocarboxylase synthetase (biotin-[proprionyl-Coenzyme A-carboxylase (ATP-hydrolysing)] ligase) | D87328 | 0.95 | 21q22.13 | Y | HLCS |
| integrin, beta 2 (antigen CD18 (p95), lymphocyte function-associated antigen 1; macrophage antigen 1 (mac-1) beta subunit) | X64072 | 0.83 | 21q22.3 | Y | ITGB2 |
| interferon (alpha, beta and omega) receptor 1 | AU137565 | 0.94 | 21q22.11 | Y | IFNAR1 |
| interferon (alpha, beta and omega) receptor 2 | L41943 | 1.28 | 21q22.11 | Y | IFNAR2 |
| interferon gamma receptor 2 (interferon gamma transducer 1) | U05875 | 1.41 | 21q22.11 | Y | IFN |
| interferon gamma receptor 2 (interferon gamma transducer 1) | U05875 | 1.41 | 21q22.11 | Y | |
| interleukin 10 receptor, beta | Z17227 | 0.97 | 21q22.11 | Y | IL10RB |
| intersectin 1 (SH3 domain protein) | AI033970 | 0.95 | 21q22.11 | Y | |
| KIAA0653 protein | AI421115 | 1.32 | 21q22.3 | | |
| minichromosome maintenance deficient (S. cerevisiae) 3-associated protein | AB011144 | 1.14 | | Y | MCM3AP |
| myxovirus (influenza) resistance 1, homolog of murine (interferon-inducible protein p78) | NM_002462 | 1.19 | 21q22.3 | Y | MX1 |
| myxovirus (influenza) resistance 2, homolog of murine | M30818 | 1.03 | 21q22.3 | N | MX2 |
| neural cell adhesion molecule 2 | U75330 | 1.07 | 21q21.1 | N | NCAM2 |
| nuclear receptor interacting protein 1 | AF248484 | 1.3 | 21q11.2 | N | NRIP1 |
| PBX/knotted 1 hoemobox 1 | Y13613 | 0.96 | 21q22.3 | Y | PKNOX1 |
| pericentrin | AB007862 | 0.93 | 21q22.3 | Y | PCNT2 |
| phosphofructokinase, liver | AL041002 | 1.29 | 21q22.3 | Y | PFKL |
| phosphoribosylglycinamide formyltransferase, phosphoribosylglycinamide synthetase, phosphoribosylaminoimidazole synthetase | AA436452 | 0:98 | 21q22.1 | | |
| pituitary tumor-transforming 1 interacting protein | BE795643 | 1.58 | 21q22.3 | Y | PTTG1IP |
| potassium inwardly-rectifying channel, subfamily J, member 15 | U73191 | 1.42 | 21q22.13 | N | KCNJ15 |
| protease, serine, 7 (enterokinase) | U09860 | 0.87 | 21q21.1 | | |
| PWP2 (periodic tryptophan protein, yeast) homolog | AP001753 | 0.95 | 21q22.3 | Y | PWP2H |
| pyridoxal (pyridoxine, vitamin B6) kinase | BE742236 | 1.5 | 21q22.3 | Y | PDXK |
| runt-related transcription factor 1 (acute myeloid leukemia 1; aml1 oncogene) | D43968 | 0.89 | 21q22.12 | Y | RUNX1 |
| S100 calcium-binding protein, beta (neural) | AV701741 | 1.21 | 21q22.3 | Y | S100B |
| SH3 domain binding glutamic acid-rich protein | BE501723 | 0.96 | 21q22.2 | N | SH3BGR |
| single-minded (Drosophila) homolog 2 | U80456 | 1.32 | 21q22.13 | Y | SIM2 |
| SMT3 (suppressor of mif two 3, yeast) homolog 1 | W55901 | 1.34 | 21q22.3 | Y | SMT3H1 |
| SON DNA binding protein | X63071 | 0.92 | 21q22.11 | | SON |
| superoxide dismutase 1, soluble (amyotrophic lateral sclerosis 1 (adult)) | AI421041 | 10.4 | 21q22.11 | Y | SOD1 |
| synaptojanin 1 | NM_003895 | 0.97 | 21q22.11 | Y | SYNJ1 |
| tetratricopeptide repeat domain 3 | D84294 | 1.23 | 21q22.13 | N | TTC3 |
| transient receptor potential channel 7 | AB001535 | 0.97 | 21q22.3 | Y | TRPM2 |
| transmembrane protease, serine 2 | U75329 | 1.16 | 21q22.3 | Y | TMPRSS2 |
| transmembrane protein 1 | U61500 | 0.95 | 21q22.3 | Y | TMEM1 |
| tryptophan rich basic protein | NM_004627 | 1.39 | 21q22.3 | Y | WRB |
| ubiquitin-conjugating enzyme E2G 2 (homologous to yeast UBC7) | AL163300 | 0.99 | 21q22.3 | Y | |
| v-ets avian erythroblastosis virus E26 oncogene homolog 2 | AF017257 | 0.98 | 21q22.2 | Y | ETS-2 |

From Table 18 above it is evident that there is variability in expression of genes of chromosome 21 in a trisomy state; some genes are highly over expressed (i.e., ratio X = 1.5; e.g., PDXK), while others are underexpressed (i.e., ratio X < 1; e.g., DSCAM). The reason for this variability can be the number of CpG sites which are methylated. Thus, for example, a 1.2 ratio suggests that not all the CpG sites in the excessive allele were subjected to methylation while those which are still methylated prevent a 1.5 fold over expression (i.e., maximal over expression of three alleles).

### EXAMPLE 5

### DSCAM and IFNAR1 genes of chromosome 21 are partially methylated in chromosome 21 trisomy

### EXAMPLE 5a

### DSCAM

The Down syndrome cell adhesion molecule (DSCAM) gene (GenBank ACCESSION NO: AF217525) was chosen to show methylation pattern of a partially silenced gene (i.e., X < 1.5) in chromosome 21 trisomy.

It was hypothesized that methylation of CpG islands upstream of DSCAM exon 1 may inhibit over expression of this gene in DS patients. The native sequence of DSCAM promoter is given in Figure 4a. A putative sequence obtained following bisulfite treatment is shown in Figure 4b.

### Experimental Procedures

***Cells*** -12days cultured amniocytes from healthy and DS affected embryos were obtained from Coriell Instiute NJ. DS cells Cat. No. GMO2067. Normal cell Cat. Nos. ***DNA extraction** -* see Example 3, above.
***Sequencing based analysis of DSCAM methylation** -* Tables 19-21 below list primers and PCR conditions which were used to amplify DSCAM from tissues and cells from healthy subjects and Down's syndrome affected subjects.

PCR reaction was effected using the primers listed in Table 19 below and the reaction mixture reagents and concentration described in Table 14 above.

**Table 19**

| Primer name | Primer Sequence (5'-3')/SEQ ID NO: | *Position (AL163283)* |
|---|---|---|
| DSCAM-f1-bis | GTTATATGGATTTTTTTGTTAATTTTTTT/87 | 333350-333379 |
| DSCAM-r1-bis | TCTCTACTACTACTTTAAAACTACAAAAC/151 | 333456-333481 |
| DSCAM-nes-f1-bis | GGTTTTAGTTATATGGATTTTTTTGTTAAT/152 | 333344-333373 |

**Table 20 - Step 1**

| ***Temperature*** | ***Time*** | ***No. Of cycles.*** |
|---|---|---|
| 94°C | 4 min | |
| 94°C | 45 sec | 35 |
| 52°C | 45 sec | |
| 72°C | 1 min | |
| 72°C | 7 min | |

| | | |
|---|---|---|
| * Reaction was effected in Buffer B-DS using primers. DSCAM-nes-f1-bis and DSCAM-r1-bis. | | |

The resultant PCR product was 142 bp.

PCR product was used as a template for a second PCR reaction (1/20 of final volume).

**Table 21- Step 2**

| ***Temperature*** | ***Time*** | ***No. of cycles*** |
|---|---|---|
| 94°C | 4 min | |
| 94°C | 45 sec | 35 |
| 53°C | 45 sec | |
| 72°C | 1 min | |
| 72°C | 7 min | |

| | | |
|---|---|---|
| Reaction was effected in Buffer NEB using primers DSCAM-fl-bis and DSCAM-r1-bis. | | |

The resultant PCR product was 135 bp. PCR reaction mixture was loaded on 3 % agarose gel and the 135 bp product was purified as described in Example 3 above. Sequence identity of the product was confirmed by sequencing as is also described hereinabove.

### Results

Sequence analysis of DSCAM methylation state in amniocytes from DS embryos showed in two cases that 25 % of the clones exhibited methylation on CpG sites. These results indicate only partial methylation of DSCAM, suggesting that the use of oligonucleotide microarrays for detecting DSCAM methylation is preferable. Oligonucleotides suitable for detecting DSCAM methylation are listed in Table 22, below.

**Table 22**

| ***WT probe (5'-3')*/ *SEQ ID NO:*** | ***Methylation probe (5'-3')*/ *SEQ ID NO:*** | ***Position in the*** ***chromosome*** ***(UCSC No.) and*** ***in AL163283 clone*** |
|---|---|---|
| tttttgtttgtgagtcgggtg/246 | tttttgtttgcgagttgggtg/153 | 41139457- |
| | | 41139477 |
| | | 333376-333396 |
| | ttttgtttgtgagtcgggtg/154 | |
| | ttttgtttgtgagttgggcg/155 | |
| | ttttgtttgcgagtcgggtg/156 | |
| | ttttgtttgcgagttgggcg/157 | |
| | ttttgtttgtgagtcgggcg/158 | |
| | ttttgtttgcgagtcgggcg/159 | |
| gtttgtgagttgggtgagtga/160 | gtttgcgagttgggtgagtga/161 | 41139462- |
| | | 41139482 |
| | | 333381-333401 |
| | gtttgtgagtcgggtgagtga/162 | |
| | gtttgtgagttgggcgagtga/163 | |
| | gtttgcgagtcgggtgagtga/164 | |
| | gtttgcgagttgggcgagtga/165 | |
| | gtttgtgagtcgggcgagtga/166 | |
| | gtttgcgagtcgggcgagtga/167 | |
| gtgagttgggtgagtgaagttg/168 | gcgagttgggtgagtgaagttg/169 | 41139475- |
| | | 41139495 |
| | | 333394-333414 |
| | gtgagtcgggtgagtgaagttg/170 | |
| | gtgagttgggcgagtgaagttg/171 | |
| | gtgagttgggtgagtgaagtcg/172 | |
| | gtgagttgggcgagtgaagtcg/173 | |
| | gtgagtcgggtgagtgaagtcg/174 | |
| | gcgagttgggtgagtgaagtcg/175 | |
| | gtgagtcgggcgagtgaagttg/176 | |
| | gcgagttgggcgagtgaagttg/177 | |
| | gcgagtcgggcgagtgaagtcg/178 | |
| | gcgagtcgggtgagtgaagttg/179 | |
| | gcgagtcgggcgagtgaagttg/180 | |
| | gcgagtcgggtgagtgaagtcg/181 | |
| | gcgagttgggcgagtgaagtcg/182 | |
| | gcgagtcgggcgagtgaagtcg/183 | |
| tgagtgaagttgagtgtggag/184 | cgagtgaagttgagtgctggag/185 | 41139476- |
| | | 41139496 |
| | | 333395-333415 |
| | tgagtgaagtcgagtgtggag/186 | |
| | tgagtgaagttgagcgtggag/187 | |
| | tgagtgaagttgagtgcggag/188 | |
| | cgagtgaagtcgagtgtggag/189 | |
| | tgagtgaagttgagcgcggag/190 | |
| | tgagtgaagtcgagtgcggag/191 | |
| | cgagtgaagttgagcgtggag/192 | |
| | tgagtgaagtcgagcgtggag/193 | |
| | cgagtgaagttgagcgtggag/194 | |
| | cgagtgaagtcgagcgtggag/195 | |
| | tgagtgaagtcgagcgcggag/196 | |
| | cgagtgaagttgagcgcggag/197 | |
| | cgagtgaagtcgagtgcggag/198 | |
| | cgagtgaagtcgagcgcggag/199 | |
| tgaagttgagtgtggaggtga/200 | tgaagtcgagtgctggaggtga/201 | 41139480- |
| | | 41139500 |
| | | 333399-333419 |
| | tgaagttgagcgtggaggtga/202 | |
| | tgaagttgagtgtggaggcga/203 | |
| | tgaagttgagtgcggaggcga/204 | |
| | tgaagttgagcgtggaggcga/205 | |
| | tgaagtcgagtgtggaggcga/206 | |
| | tgaagttgagcgcggaggtga/207 | |
| | tgaagtcgagtgcggaggtga/208 | |
| | tgaagtcgagcgtggaggtga/209 | |
| | tgaagtcgagcgcggaggtga/210 | |
| | tgaagtcgagcgtggaggcga/211 | |
| | tgaagtcgagtgcggaggcga/212 | |
| | tgaagttgagcgcggaggcga/213 | |
| | tgaagtcgagcgcggaggcga/214 | |
| aagttgagtgtggaggtgagt/215 | aagtcgagtgctggaggtgagt/216 | 41139481- |
| | | 41139501 |
| | | 333401-333421 |
| | aagttgagcgtggaggtgagt/217 | |
| | aagttgagtgtggaggcgagt/218 | |
| | aagttgagtgcggaggcgagt/219 | |
| | aagttgagcgtggaggcgagt/220 | |
| | aagtcgagtgtggaggcgagt/221 | |
| | aagttgagcgcggaggtgagt/222 | |
| | aagtcgagtgcggaggtgagt/223 | |
| | aagtcgagcgtggaggtgagt/224 | |
| | aagtcgagcgcggaggtgagt/225 | |
| | aagtcgagcgtggaggcgagt/226 | |
| | aagtcgagtgcggaggcgagt/227 | |
| | aagttgagcgcggaggcgagt/228 | |
| | aagtcgagcgcggaggcgagt/229 | |
| agtgtggaggtgagtagggat/230 | gtgcggaggtgagtagggat/231 | 41139488- |
| | | 41139508 |
| | | 333407-333427 |
| | gcgtggaggtgagtagggat/232 | |
| | gtgtggaggcgagtagggat/233 | |
| | gtgcggaggcgagtagggat/234 | |
| | gcgtggaggcgagtagggat/235 | |
| | gcgcggaggtgagtagggat/236 | |
| | gcgcggaggcgagtagggat/237 | |
| tgtttttggttgttggggtgt/238 | tgtttttggtcgttggggtgt/239 | 41139517- |
| | | 41139537 |
| | | /333436-333456 |
| | tgtttttggttgttggggcgt/240 | |
| | tgtttttggtcgttggggcgt/241 | |
| gttgttggggtgttttgtagt/242 | gtcgttggggtgttttgtagt/243 | 41139525- |
| | | 41139545 |
| | | 333444-333464 |
| | gttgttggggcgttttgtagt/244 | |
| | gtcgttggggcgttttgtagt/245 | |

### EXAMPLE 5b

### IFNAR1

From Table 18 above it is evident that Interferon (alpha, beta and omega) Receptor 1 (IFNAR1, GenBank Accession No: AU137565) is partially silenced in chromosome 21 trisomy. The methylation pattern of IFNAR1 was examined in cells and tissues as described in Example 5a. The native sequence of IFNAR1 promoter is given in Figure 5a. A putative sequence obtained following bisulfite treatment is shown in Figure 5b.

### Experimental Procedures

### Cells - See above.

***DNA extraction** -* see Example 3, above.

***Sequencing based analysis of DSCAM methylation -*** Tables 23-25 below list primers and PCR conditions which were used to amplify IFNAR1 from tissues and cells from healthy subjects and Down's syndrome affected subjects.

PCR reaction was effected using the primers listed in Table 23 below and the reaction mixture reagents and concentration described in Table 14 above.

**Table 23**

| ***Primer name*** | ***Position in (AY654286)*** | ***Sequence (5'-3')*/*SEQ ID NO:*** |
|---|---|---|
| IFNR-f4-bis | 1327-1351 | TTTTAGTTTTATTTGGTTTTTAGGT/247 |
| IFNR-r4-bis | 1372-1396 | AAAAAACCTTAACCTTCACAAAATC/248 |
| IFNR-nes-f-bis | 1533-1557 | ATTGTTTAAGATTTTAGGGTTAGTA/ 249 |

**Table 24 - Step 1**

| ***Temperature*** | ***Time*** | ***No. of Cycles*** |
|---|---|---|
| 94°C | 4 min | |
| 94°C | 45 sec | 35 |
| 54°C | 45 sec | |
| 72°C | 1 min | |
| 72°C | 7 min | |

| | | |
|---|---|---|
| Reaction was effected in Buffer B-DS using primers IFNR-f4-bis and IFNR-r4-bis. | | |

The resultant PCR product was 231 bp.

PCR product was used as a template for a second PCR reaction (1/20 of final volume).

**Table 25 - Step 2**

| ***Temperature*** | ***Time*** | ***No. of Cycles*** |
|---|---|---|
| 94°C | 4 min | |
| 94°C | 45 sec | 35 |
| 56°C | 45 sec | |
| 72°C | 1 min | |
| 72°C | 7 min | |

| | | |
|---|---|---|
| Reaction was effected in Buffer NEB using primers IFNR-nes-f-bis and IFNR-r4-bis | | |

The resultant PCR product was 186 bp.

PCR reaction products were resolved on 2.5 % agarose gel and the 186 bp product was purified as described in Example 3 above. Sequence identity of the product was confirmed by sequencing as is also described hereinabove.

### Results

Methylation of IFNAR1 alleles was seen in DS samples.

From the above described, it is conceivable that DSCAM and IFNAR1 methylation state can serve as valuable diagnostic markers for chromosome 21 trisomy. These results also indicate that other genes which are not upregulated in chromosome 21 trisomy can serve as markers for chromosome amplification as well.

### EXAMPLE 6

### Putative markers for Chromosome 13 autosomal trisomy

Table 26 below, shows ratio of chromosome 13 gene expression in amniotic cells obtained from trisomy 13 genotyped subjects versus amniotic cells obtained from normal subjects (www.hgu.mrc.ac.uk/Research/Cellgen/Supplements/Unigene/t13all.htm.). Interestingly, contrary to chromosome 21 trisomy where most genes are silenced (RNA is insteady state levels), this profile of gene expression does not occur in chromosome 13, explaining the vitality of chromosome 21 amplification.

**Table 26**

| Gene Name | Accession No. | Ratio | Location |
|---|---|---|---|
| ADP-ribosyltransferase (NAD+; poly (ADP-ribose) polymerase)-like 1 | NM_006437 | 1.6 | 13q34 |
| ATPase, H+/K+ exchanging, beta polypeptide | NM_000705 | 1.11 | 13q34 |
| carboxypeptidase B2 (plasma) | NM_001872 | 0.92 | 13q14.13 |
| CDC16 (cell division cycle 16, S. cerevisiae, homolog) | NM_003903 | 1.08 | 13q34 |
| ceroid-lipofuscinosis, neuronal 5 | NM_006493 | 1.5 | 13q22.3 |
| coagulation factor X | AL521984 | 1.09 | 13q34 |
| collagen, type IV, alpha 1 | XM_007094 | 0.43 | 13q34 |
| cullin 4A | AI638597 | 1.58 | 13q34 |
| cyclin A1 | NM_003914 | 1.06 | 13q13.3 |
| cyclin-dependent kinase 8 | BE467537 | 1.59 | 13q12 |
| dachshund (Drosophila) homolog | NM_004392 | 1.69 | 13q21.33 |
| DnaJ (Hsp40) homolog, subfamily C, member 3 | AW772531 | 1.22 | 13q32.1 |
| doublecortin and CaM kinase-like 1 | NM_004734 | 1.4 | 13q13.3 |
| endothelin receptor type B | BE837728 | 1 | 13q22.3 |
| excision repair cross-complementing rodent repair deficiency, complementation group 5 (xeroderma pigmentosum, complementation group G (Cockayne syndrome)) | NM_000123 | 1.12 | 13q33.1 |
| FERM, RhoGEF (ARHGEF) and pleckstrin domain protein 1 (chondrocyte-derived) | BF793662 | 1.19 | 13q32.2 |
| fibroblast growth factor 9 (glia-activating factor) | AI869879 | 0.98 | 13q12.11 |
| fms-related tyrosine kinase 1 (vascular endothelial growth factor/vascular permeability factor receptor) | NM_002019 | 1.73 | 13q12.3 |
| fms-related tyrosine kinase 1 (vascular endothelial growth factor/vascular permeability factor receptor) | NM_002019 | 1.73 | 13q12.3 |
| fms-related tyrosine kinase 3 | NM_004119 | 1.3 | 13q12.2 |
| forkhead box O1A (rhabdomyosarcoma) | NM_002015 | 1.17 | 13q14.11 |
| growth arrest-specific 6 | NM_000820 | 0.76 | 13q34 |
| Human BRCA2 region, mRNA sequence CG011 | U50536 | 0.67 | 13q13.1 |
| inhibitor of growth 1 family, member 1 | AF181850 | 0.99 | 13q34 |
| integrin, beta-like 1 (with EGF-like repeat domains) | NM_004791 | 0.68 | 13q33.1 |
| karyopherin alpha 3 (importin alpha 4) | NM_002267 | 1.11 | 13q14.2 |
| klotho | NM_004795 | 1.44 | 13q13.1 |
| ligase IV, DNA, ATP-dependent | NM_002312 | 1.3 | 13q33.3 |
| lipoma HMGIC fusion partner | N67270 | 1.05 | 13q13.3 |
| lymphocyte cytosolic protein 1 (L-plastin) | BF035921 | 0.98 | 13q14.13 |
| mitochondrial intermediate peptidase | AA524277 | 0.88 | 13q12.12 |
| mitochondrial translational release factor 1 | AI884353 | 0.99 | 13q14.11 |
| myotubularin related protein 6 | AW205652 | 1.63 | 13q12.13 |
| osteoblast specific factor 2 (fasciclin I-like) | N71912 | 1.91 | 13q13.3 |
| peroxiredoxin 2 | AL523978 | 1.11 | |
| propionyl Coenzyme A carboxylase, alpha polypeptide | NM_000282 | 1.22 | 13q32.3 |
| protein phosphatase 1, regulatory (inhibitor) subunit 2 | AI141349 | 1.57 | |
| purinergic receptor (family A group 5) | AI823889 | 1.25 | 13q14.2 |
| replication factor C (activator 1) 3 (38kD) | AA907044 | 0.96 | 13q13.2 |
| ret finger protein 2 | AL526890 | 1.25 | 13q14.2 |
| (retinoblastoma 1 (including osteosarcoma) | NM_000321 | 1.65 | 13q14.2 |
| sciellin | AK025320 | 0.95 | 13q22.3 |
| serine/threonine kinase 24 (Ste20, yeast homolog) | NM_003576 | 1.12 | |
| serine/threonine kinase 24 (Ste20, yeast homolog) | AU146392 | 1.06 | 13q32.2 |
| solute carrier family 10 (sodium/bile acid cotransporter family), member 2 | NM_000452 | 1.32 | 13q33.1 |
| solute carrier family 25 (mitochondrial carrier; ornithine transporter) member 15 | AI382550 | 0.87 | 13q14.11 |
| solute carrier family 7 (cationic amino acid transporter, y+ system), member 1 | X57303 | 1.09 | 13q12.3 |
| spastic ataxia of Charlevoix-Saguenay (sacsin) | AB018273 | 0.97 | 13q12.12 |
| sprouty (Drosophila) homolog 2 | NM_005842 | 1.54 | 13q31.1 |
| transcription factor Dp-1 | NM_007111 | 1.23 | 13q34 |
| transmembrane 9 superfamily member 2 | AU131084 | 0.97 | 13q32.3 |
| tripeptidyl peptidase II | NM_003291 | 1.1 | 13q33.1 |
| tumor necrosis factor (ligand) superfamily, member 11 | AF053712 | 1.14 | 13q14.11 |
| Zic family member 2 (odd-paired Drosophila homolog) | AF188733 | 1.9 | 13q32.3 |
| zinc finger protein 198 | AL138688 | 1.45 | 13q12.11 |

### EXAMPLE 7

### Genes of trisomy 9 and primers which can be used for detecting methylation status thereof

Trisomy 9 is a rare chromosomal disorder. Characteristic features include delayed growth of the fetus, heart defects present at birth, facial abnormalities (e.g., low-set and/or malformed ears), an abnormally small head, kidney and/or genital abnormalities, skeletal abnormalities (e.g., fixed and/or dislocated joints), and/or malformations of the brain.

p16 on chromosome 9 plays a central role in cell cycle and in many pathologies including melanoma, bladder and lung cancer. Expression of p16, a tumor suppressor gene, is repressed in a variety of cancers such as bladder, colon and retinoblastoma. Methylation of CpG islands in the p16 promoter has been shown to be responsible for inactivation of this gene in certain cases [Sharpless (2003) Oncogene. 22(20):3092-8; Virmani (2003) Methods Mol Biol. 2003;222:97-115].

The CpG WIZ® p 16 Amplification Kit (Chemicon International, Inc.) is used for determining the methylation status of the p16 promoter by methylation-specific PCR (MSP). The kit contains primers targeted to regions of the promoter where the sequences are most divergent following bisulfite treatment. PCR parameters have been identified such that all primer sets in the kit amplify under the same conditions. Control genomic DNA samples (methylated and unmethylated) for p16 are also included.

### Experimental Procedures

Bisulfite conversion is carried out using the CpGenome DNA Modification Kit (Intergen, New York, NY). 1 µg of DNA is treated with sodium bisulfite according to manufacturers recommendations. Following conversion, the bisulfite-treated DNA is resuspended in a total volume of 25 µl.

Table 27 below summarizes the methods which are used to detect methylation state of the above-described genes.

**Table 27**

| ***Method*** | ***Example*** | ***Trisomy*** |
|---|---|---|
| DNA Sequencing | *APP,AR. p16,DSCAM BACHI ETS2 INFAR1 | 21,X,9 |
| Restriction enzyme | Androgen Receptor | X |
| MSP | Androgen Receptor | X, |
| Microarray | APP | 21,X |
| Commercial kit for mutation's detection | p16 | 9 |

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

## Claims

1. A method of identifying locus amplification, the method comprising determining a methylation state of at least one gene in the locus, said gene being selected having an expression pattern which is compatible with two gene copies, wherein an increase in methylation state of said at least one gene in the locus compared to a methylation state of said at least one gene in a non-amplified locus is indicative of locus amplification.

2. A method of identifying locus amplification in a subject, the method comprising:
determining a methylation state of at least one gene at the locus in a chromosomal DNA of the subject, said gene being selected having an expression pattern which is compatible with two gene copies, wherein an increase in methylation state of said at least one gene in the locus compared to a methylation state of said at least one gene in a non-amplified locus is indicative of locus amplification in the subject.

3. A method of prenatally identifying locus amplification, the method comprising:
determining a methylation state of at least one gene at the locus in a prenatal chromosomal DNA, said gene being selected having an expression pattern which is compatible with two gene copies, wherein an increase in methylation state of said at least one gene in the locus compared to a methylation state of said at least one gene in a non-amplified locus is indicative of locus amplification in the prenatal subject.

4. A method of prenatally testing Down's syndrome, the method comprising determining a methylation state of at least one gene in a prenatal chromosome 21, wherein said at least one gene is selected having an expression pattern which is compatible with two gene copies and whereas an increase in a state of said methylation of said at least one gene compared to a methylation state of said at least one gene in a non-amplified locus is indicative of amplification of said at least one gene, thereby prenatally diagnosing Down's syndrome.

5. The method of claim 1, 2, 3 or 4, wherein said determining methylation state of said at least one gene is effected by:
(i) restriction enzyme digestion methylation detection;
(ii) bisulphate-based methylation detection;
(iii) mass-spectrometry analysis;
(iv) sequence analysis; and/or
(v) microarray analysis.

6. The method of claim 1, 2 or 3, wherein the locus is located on a chromosome selected from the group consisting of chromosome 1, chromosome 2, chromosome 3, chromosome 4, chromosome 5, chromosome 6, chromosome 7, chromosome 8, chromosome 9, chromosome 10, chromosome 11, chromosome 12, chromosome 13, chromosome 14, chromosome 15, chromosome 16, chromosome 17, chromosome 18, chromosome 19, chromosome 20, chromosome 21, chromosome 22, chromosome X and chromosome Y.

7. The method of claim 3 or 4, wherein prenatal chromosomal DNA is obtained by:
(i) amniocentesis;
(ii) fetal biopsy;
(iii) chorionic villi sampling;
(iv) maternal biopsy;
(v) blood sampling; and/or
(vi) cervical sampling.

8. The method of claim 4, wherein said at least one gene is selected from the group consisting of APP, cystathionine-β-synthase, amyloid beta (A4) precursor protein (protease nexin-II, Alzheimer disease), ATP-binding cassette, sub-family G (WHITE), member 1, autoimmune regulator (automimmune polyendocrinopathy candidiasis ectodermal dystrophy), BTB and CNC homology 1, basic leucine zipper transcription factor 1, BTG family, member 3, carbonyl reductase 1, carbonyl reductase 3, chromatin assembly factor 1, subunit B (p60), chromosome 21 open reading frame 18, chromosome 21 open reading frame 18, chromosome 21 open reading frame 2, collagen, type VI, alpha 1, collagen, type VI, alpha 2, collagen, type XVIII, alpha 1, coxsackie virus and adenovirus receptor, cystatin B (stefin B), DNA segment on chromosome 21 (unique) 2056 expressed sequence, Down syndrome cell adhesion molecule, Down syndrome critical region gene 1, Down syndrome critical region gene 3, f-box and WD-40 domain protein 1B, glutamate receptor, ionotropic, kainate 1, HMT1 (hnRNP methyltransferase, S. cerevisiae)-like 1, holocarboxylase synthetase (biotin-[proprionyl-Coenzyme A-carboxylase (ATP-hydrolysing)] ligase), integrin, beta 2 (antigen CD18 (p95), lymphocyte function-associated antigen 1; macrophage antigen 1 (mac-1) beta subunit), interferon (alpha, beta and omega) receptor 1, interferon (alpha, beta and omega) receptor 2, interferon gamma receptor 2 (interferon gamma transducer 1), interferon gamma receptor 2 (interferon gamma transducer 1), interleukin 10 receptor, beta, intersectin 1 (SH3 domain protein), KIAA0653 protein, minichromosome maintenance deficient (S. cerevisiae) 3-associated protein, myxovirus (influenza) resistance 1, homolog of murine (interferon-inducible protein p78), myxovirus (influenza) resistance 2, homolog of murine, neural cell adhesion molecule 2, nuclear receptor interacting protein 1, PBX/knotted 1 hoemobox 1, pericentrin, phosphofructokinase, liver, phosphoribosylglycinamide formyltransferase, phosphoribosylglycinamide synthetase, phosphoribosylaminoimidazole synthetase, pituitary tumor-transforming 1 interacting protein, potassium inwardly-rectifying channel, subfamily J, member 15, protease, serine, 7 (enterokinase), PWP2 (periodic tryptophan protein, yeast) homolog, pyridoxal (pyridoxine, vitamin B6) kinase, runt-related transcription factor 1 (acute myeloid leukemia 1; aml1 oncogene), S100 calcium-binding protein, beta (neural), SH3 domain binding glutamic acid-rich protein, single-minded (Drosophila) homolog 2, SMT3 (suppressor of mif two 3, yeast) homolog 1, SON DNA binding protein, superoxide dismutase 1, soluble (amyotrophic lateral sclerosis 1 (adult)), synaptojanin 1, tetratricopeptide repeat domain 3, transient receptor potential channel 7, transmembrane protease, serine 2, transmembrane protein 1, tryptophan rich basic protein, ubiquitin-conjugating enzyme E2G 2 (homologous to yeast UBC7), v-ets avian erythroblastosis virus E26 oncogene homolog 2.

9. The method of claim 4, said at least one gene being selected from the group consisting of APP and cystathionine-β-synthase.

## Patentansprüche

1. Verfahren zum Identifizieren einer Locusamplifikation, wobei das Verfahren das Bestimmen eines Methylierungszustands von mindestens einem Gen in dem Locus umfasst, wobei das Gen mit einem Expressionsmuster ausgewählt wird, das mit zwei Genkopien kompatibel ist, wobei eine Zunahme des Methylierungszustands des mindestens einen Gens in dem Locus im Vergleich zu einem Methylierungszustand des mindestens einen Gens in einem nicht-amplifizierten Locus eine Locusamplifikation anzeigt.

2. Verfahren zum Identifizieren einer Locusamplifikation in einem Versuchsobjekt, wobei das Verfahren umfasst:
Bestimmen eines Methylierungszustands von mindestens einem Gen an dem Locus in einer chromosomalen DNA des Versuchsobjekts, wobei das Gen mit einem Expressionsmuster ausgewählt wird, das mit zwei Genkopien kompatibel ist, wobei eine Zunahme des Methylierungszustands des mindestens einen Gens in dem Locus im Vergleich zu einem Methylierungszustand des mindestens einen Gens in einem nicht-amplifizierten Locus eine Locusamplifikation in dem Versuchsobjekt anzeigt.

3. Verfahren zum pränatalen Identifizieren einer Locusamplifikation, wobei das Verfahren umfasst:
Bestimmen eines Methylierungszustands von mindestens einem Gen am Locus in einer pränatalen chromosomalen DNA, wobei das Gen mit einem Expressionsmuster ausgewählt wird, das mit zwei Genkopien kompatibel ist, wobei eine Zunahme des Methylierungszustands des mindestens einen Gens in dem Locus im Vergleich zu einem Methylierungszustand des mindestens einen Gens in einem nicht-amplifizierten Locus eine Locusamplifikation in dem pränatalen Versuchsobjekt anzeigt.

4. Verfahren zum pränatalen Testen auf Downsches Syndrom, wobei das Verfahren das Bestimmen eines Methylierungszustands von mindestens einem Gen in einem pränatalen Chromosom 21 umfasst, wobei das mindestens eine Gen mit einem Expressionsmuster ausgewählt wird, das mit zwei Genkopien kompatibel ist, und wobei eine Zunahme eines Methylierungszustands des mindestens einen Gens im Vergleich zu einem Methylierungszustand des mindestens einen Gens in einem nicht-amplifizierten Locus eine Amplifikation des mindestens einen Gens anzeigt, wodurch das Down-Syndrom pränatal diagnostiziert wird.

5. Verfahren nach Anspruch 1, 2, 3 oder 4, wobei das Bestimmen des Methylierungszustands des mindestens einen Gens bewirkt wird durch:
(i) Methylierungsnachweis durch Restriktionsenzymverdau;
(ii) Methylierungsnachweis auf Bisulfat-Basis;
(iii) Massenspektrometrieanalyse;
(iv) Sequenzanalyse; und/oder
(v) Microarray-Analyse.

6. Verfahren nach Anspruch 1, 2 oder 3, wobei der Locus auf einem Chromosom lokalisiert ist, ausgewählt aus der Gruppe, bestehend aus Chromosom 1, Chromosom 2, Chromosom 3, Chromosom 4, Chromosom 5, Chromosom 6, Chromosom 7, Chromosom 8, Chromosom 9, Chromosom 10, Chromosom 11, Chromosom 12, Chromosom 13, Chromosom 14, Chromosom 15, Chromosom 16, Chromosom 17, Chromosom 18, Chromosom 19, Chromosom 20, Chromosom 21, Chromosom 22, Chromosom X und Chromosom Y.

7. Verfahren nach Anspruch 3 oder 4, wobei die pränatale chromosomale DNA erhalten wird durch:
(i) Amniozentese;
(ii) fetale Biopsie;
(iii) Chorionzotten-Probenentnahme;
(iv) Biopsie der Mutter;
(v) Blutprobenahme;
(vi) Zervikalprobenahme.

8. Verfahren nach Anspruch 4, wobei das mindestens eine Gen ausgewählt ist aus der Gruppe, bestehend aus APP, Cystathionin-β-Synthase, Amyloid-beta-(A4)-Vorläuferprotein (Protease Nexin-II, Alzheimer-Krankheit), ATP-bindende Kassette, Unterfamilie G (WHITE), Member 1, Autoimmunregulator (polyendokrine Autoimmunerkrankung Candidiasis-ektodermale Dystrophie), BTB- und CNC-Homologie 1, Basic-Leucine-Zipper-Transkriptionsfaktor 1, BTG-Familie, Member 3, Carbonylreduktase 1, Carbonylreduktase 3, Chromatin-Assembly-Faktor 1, Untereinheit B (p60), Chromosom 21 offener Leserahmen 18, Chromosom 21 offener Leserahmen 2, Kollagen, Typ VI, Alpha 1, Kollagen, Typ VI, Alpha 2, Kollagen, Typ XVIII, Alpha 1, Coxsackie-Virus- und Adenovirus-Rezeptor, Cystatin B (Stefin B), DNA-Segment auf Chromosom 21 (einmalig) 2056 exprimierte Sequenz, Down-Syndrom-Zelladhäsionsmolekül, Down-Syndrom-kritische Region Gen 1, Down-Syndrom-kritische Region Gen 3, F-Box- und WD-40-Domäne-Protein 1B, Glutamat-Rezeptor, ionotrop, Kainat 1, HMT1 (hnRNP-Methyltransferase, S. cerevisiae)-ähnlich 1, Holocarboxylase-Synthetase (Biatin-[Proprionyl-Coenzym-A-Carboxylase-(ATP-hydrolysierende)]-Ligase); Integrin, Beta 2 (Antigen CD18 (p95), Lymphozytenfunktionassoziiertes Antigen 1; Makrophagen-Antigen 1 (Mac-1) Beta-Untereinheit), Interferon-(alpha-, beta- und omega-)-Rezeptor 2, Interferon-gamma-Rezeptor 2 (Interferon-gamma-Transducer 1), Interleukin-10-Rezeptor, Beta, Intersectin 1 (SH3-Domäne-Protein), KIAA0653-Protein, Minichromosom-Maintenancedefizientes (S. cerevisiae)-3-assoziiertes Protein, Myxovirus-(Influenza)-Resistenz 1, Homolog von murinem (Interferon-induzierbarem) Protein p78, Myxovirus-(Influenza)-Resistenz 2, Homolog von murinem Neuralzellenadhäsionsmolekül 2, Kernrezeptor-interagierendes Protein 1, PB3X/Knotted 1 Homeobox 1, Pericentrin, Phosphofructokinase, Leber, Phosphoribosylglycinamidformyltransferase, Phosphoribosylglycinamid-Synthetase, Phosphoribosylaminoimidazol-Synthetase, Hypophysentumortransformierendes 1-interagierendes Protein, Potassium-Inwardly-Rectifying-Channel, Unterfamilie J, Member 15, Protease, Serin, 7 (Enterokinase), PWP2-(periodisches Tryptophanprotein, Hefe)-Homolog, Pyridoxal-(Pyridoxin, Vitamin B6)-Kinase, Runt-Related-Transkriptionsfaktor 1 (akute myeloische Leukämie 1; AML1-Onkogen), S100-Calcium-bindendes Protein, Beta (neural), SH3-Domäne-bindendes glutaminsäurereiches Protein, Single-Minded-(Drosophila)-Homolog 2, SMT3 (Suppressor von mif two 3, Hefe)-Homolog 1, SON-DNA-Bindungsprotein, Superoxiddismutase 1, löslich (amyotrophische Lateralsklerose-1-(adult)), Synaptojanin 1, Tetratricopeptid-Repeat-Domäne 3, TRPC-Kanal (Transient Receptor Potential Channel) 7, Transmembranprotease, Serin 2, Transmembranprotein 1, tryptophanreiches basisches Protein, Ubiquitin-konjugierendes Enzym E2G 2 (homolog zu Hefe UBC7), V-ets-Vogel-Erythroblastose-Virus E26-Onkogen-Homolog 2.

9. Verfahren nach Anspruch 4, wobei das mindestens eine Gen ausgewählt ist aus der Gruppe, bestehend aus APP und Cystathionin-β-Synthase.

## Revendications

1. Procédé d'identification d'une amplification de locus, le procédé comprenant la détermination d'un état de méthylation d'au moins un gène dans le locus, ledit gène étant choisi ayant un motif d'expression qui est compatible avec deux copies de gène, une augmentation de l'état de méthylation dudit au moins un gène dans le locus par comparaison avec un état de méthylation dudit au moins un gène dans un locus non amplifié étant indicative d'une amplification de locus.

2. Procédé d'identification d'une amplification de locus dans un sujet, le procédé comprenant :
la détermination d'un état de méthylation d'au moins un gène au niveau du locus dans un ADN chromosomique du sujet, ledit gène étant choisi ayant un motif d'expression qui est compatible avec deux copies de gène, une augmentation de l'état de méthylation dudit au moins un gène dans le locus par comparaison avec un état de méthylation dudit au moins un gène dans un locus non amplifié étant indicative d'une amplification de locus dans le sujet.

3. Procédé d'identification prénatale d'une amplification de locus, le procédé comprenant :
la détermination d'un état de méthylation d'au moins un gène au niveau du locus dans un ADN chromosomique prénatal, ledit gène étant choisi ayant un motif d'expression qui est compatible avec deux copies de gène, une augmentation de l'état de méthylation dudit au moins un gène dans le locus par comparaison avec un état de méthylation dudit au moins un gène dans un locus non amplifié étant indicative d'une amplification de locus dans le sujet prénatal.

4. Procédé de test prénatal du syndrome de Down, le procédé comprenant la détermination d'un état de méthylation d'au moins un gène dans un chromosome 21 prénatal, dans lequel ledit au moins un gène est choisi ayant un motif d'expression qui est compatible avec deux copies de gène et dans lequel une augmentation d'un état de ladite méthylation dudit au moins un gène par comparaison avec un état de méthylation dudit au moins un gène dans un locus non amplifié est indicative d'une amplification dudit au moins un gène, permettant ainsi de diagnostiquer de façon prénatale le syndrome de Down.

5. Procédé selon l'une des revendications 1, 2, 3 ou 4, dans lequel ladite détermination de l'état de méthylation dudit au moins un gène est effectuée par :
(i) détection de méthylation par digestion par enzyme de restriction ;
(ii) détection de methylation base de bisulfate ;
(iii) analyse par spectrométrie de masse ;
(iv) analyse de séquence ; et/ou
(v) analyse de microréseau.

6. Procédé selon l'une des revendications 1, 2 ou 3, dans lequel le locus est situé sur un chromosome choisi dans le groupe constitué par le chromosome 1, le chromosome 2, le chromosome 3, le chromosome 4, le chromosome 5, le chromosome 6, le chromosome 7, le chromosome 8, le chromosome 9, le chromosome 10, le chromosome 11, le chromosome 12, le chromosome 13, le chromosome 14, le chromosome 15, le chromosome 16, le chromosome 17, le chromosome 18, le chromosome 19, le chromosome 20, le chromosome 21, le chromosome 22, le chromosome X et le chromosome Y.

7. Procédé selon l'une des revendications 3 ou 4, dans lequel l'ADN chromosomique prénatal est obtenu par :
(i) amniocentése ;
(ii) biopsie foetale ;
(iii) prélèvement des villosités choriales ;
(iv) biopsie maternelle ;
(v) prise de sang ; et/ou
(vi) prélèvement cervical.

8. Procédé selon la revendication 4, dans lequel ledit au moins un gène est choisi dans le groupe constitué par l'APP, la cystathionine-β-synthase, la protéine précurseur de l'amyloïde bêta (A4) (protéase nexine-II, maladie d'Alzheimer), cassette de liaison à l'ATP, sous-famille G (WHITE), élément 1, régulateur auto-immun (dystrophie ectodermique de candidose de polyendocrinopathie auto-immune), homologie 1 de BTB et CNC, facteur de transcription de tirette à leucine basique 1, famille BTG, élément 3, carbonyl réductase 1, carbonyl réductase 3, facteur d'assemblage de la chromatine 1, sous-unité B (p60), cadre de lecture ouvert 18 du chromosome 21, cadre de lecture ouvert 2 du chromosome 21, collagène, type VI, alpha 1, collagène, type VI, alpha 2, collagène, type XVIII, alpha 1, virus Coxsackie et récepteur d'adénovirus, cystatine B (stéfine B), segment d'ADN sur la séquence exprimée 2056 du chromosome 21 (unique), molécule d'adhésion à la cellule du syndrome de Down, gène 1 de région critique du syndrome de Down, gène 3 de région critique du syndrome de Down, f-box et protéine 1B du domaine WD-40, récepteur de glutamate, ionotropique, kainate 1, analogue 1 de HMT1 (hnRNP méthyltransférase, S. cerivisiae), holocarboxylase synthétase (biotine-[proprionyl-Coenzyme A-carboxylase (hydrolysant l'ATP)] ligase), intégrine, bêta 2 (antigène CD18 (p95), antigène 1 associe à la fonction des lymphocytes ; sous-unité bêta de l'antigène 1 de macrophage (mac-1)), récepteur 1 d'interféron (alpha, bêta et oméga), récepteur 2 d'interféron (alpha, bêta et oméga), récepteur 2 d'interféron gamma (transducteur 1 d'interféron gamma), récepteur d'interleukine 10, bêta, intersectine 1 (protéine du domaine SH3), protéine KIAA0653, protéine 3 associée à une déficience d'entretien de minichromosome (S. cerivisiae), résistance 1 aux mixovirus (grippe), homologue de murine (protéine p78 inductible par l'interféron), résistance 2 aux myxovirus (grippe), homologue de murine, molécule d'adhésion aux cellules neurales 2, protéine d'interaction aux récepteurs nucléaires 1, PBX/knotted 1 homeobox 1, péricentrine, phosphofructokinase, foie, phosphoribosylglycinamide formyltransférase, phosphoribosylglycinamide synthétise, phosphoribosylaminoimidazole synthétise, protéine d'interaction 1 de transformation de la tumeur pituitaire, canal de rectification interne du potassium, sous-famille J, élément 15, protéase, sérine, 7 (entérokinase), homologue de PWP2 (protéine de tryptophane périodique, levure), pyridoxal (pyridoxine, vitamine B6) kinase, facteur de transcription apparenté à runt-1 (leucémie myéloïde aiguë 1 ; oncogène ami 1), protéine de liaison au calcium S100, bêta (neural), protéine riche en acide glutamique se liant au domaine SH3, homologue 2 à simple élément (Drosophila), homologue 1 de SMT3 (suppresseur de mif deux 3, levure), protéine de liaison à l'ADN SON, superoxyde dismutase 1, soluble (sclérose latérale amyotrophique 1 (adulte)), synaptojanine 1, domaine de répétition de tétratricopeptide 3, canal potentiel de récepteur transitoire 7, protéase transmembranaire, sérine 2, protéine transmembranaire 1, protéine basique riche en tryptophane, E2G 2 enzyme de conjugaison à l'ubiquitine (homologue à la levure Ubac7), homologue 2 de l'oncogène du virus E26 de l'érythroblastose aviaire v-ets.

9. Procédé selon la revendication 4, dans lequel ledit au moins un gène est choisi dans le groupe constitué par l'APP et la cystathionine-β-synthase.


### SEQUENCE LISTING

<110> Halle, David
<120> METHODS AND KITS USEFUL FOR DETECTING AN ALTERATION IN A LOCUS COPY NUMBER
<130> 28110
<160> 249
<170> PatentIn version 3.2
<210> 1
   <211> 25
   <212> DNA
   <213> Artificial sequence
<220>
   <223> Single strand DNA oligonucleotide
<400> 1
   tggttttaga tttttttttt tattg 25
<210> 2
   <211> 25
   <212> DNA
   <213> Artificial sequence
<220>
   <223> Single strand DNA oligonucleotide
<400> 2
   acctaccact accaaaaaaa ctaac 25
<210> 3
   <211> 15
   <212> DNA
   <213> Artificial sequence
<220>
   <223> Single strand DNA oligonucleotide
<400> 3
   tattttttgg tgtta 15
<210> 4
   <211> 15
   <212> DNA
   <213> Artificial sequence
<220>
   <223> Single strand DNA oligonucleotide
<400> 4
   tatttttcgg tgtta 15
<210> 5
   <211> 15
   <212> DNA
   <213> Artificial sequence
<220>
   <223> Single strand DNA oligonucleotide
<400> 5
   gagggggtgt gtggg 15
<210> 6
   <211> 15
   <212> DNA
   <213> Artificial sequence
<220>
   <223> Single strand DNA oligonucleotide
<400> 6
   gagggggcgt gtggg 15
<210> 7
   <211> 15
   <212> DNA
   <213> Artificial sequence
<220>
   <223> Single strand DNA oligonucleotide
<400> 7
   gttaaggtgt tgtat 15
<210> 8
   <211> 15
   <212> DNA
   <213> Artificial sequence
<220>
   <223> Single strand DNA oligonucleotide
<400> 8
   gttaaggcgt tgtat 15
<210> 9
   <211> 15
   <212> DNA
   <213> Artificial sequence
<220>
   <223> Single strand DNA oligonucleotide
<400> 9
   ttgtgggtgt ggggt 15
<210> 10
   <211> 15
   <212> DNA
   <213> Artificial sequence
<220>
   <223> Single strand DNA oligonucleotide
<400> 10
   ttgtgggcgt ggggt 15
<210> 11
   <211> 15
   <212> DNA
   <213> Artificial sequence
<220>
   <223> Single strand DNA oligonucleotide
<400> 11
   tttttggtgt gagtg 15
<210> 12
   <211> 15
   <212> DNA
   <213> Artificial sequence
<220>
   <223> Single strand DNA oligonucleotide
<400> 12
   tttttggcgt gagtg 15
<210> 13
   <211> 15
   <212> DNA
   <213> Artificial sequence
<220>
   <223> Single strand DNA oligonucleotide
<400> 13
   gagtgggtgt agttt 15
<210> 14
   <211> 15
   <212> DNA
   <213> Artificial sequence
<220>
   <223> Single strand DNA oligonucleotide
<400> 14
   gagtgggcgt agttt 15
<210> 15
   <211> 16
   <212> DNA
   <213> Artificial sequence
<220>
   <223> Single strand DNA oligonucleotide
<400> 15
   tttggtggtg ttgtta 16
<210> 16
   <211> 16
   <212> DNA
   <213> Artificial sequence
<220>
   <223> Single strand DNA oligonucleotide
<400> 16
   tttggtggcg ttgtta 16
<210> 17
   <211> 16
   **<212>** DNA
   <213> Artificial sequence
<220>
   <223> Single strand DNA oligonucleotide
<400> 17
   ggttgttgtg tttggg 16
<210> 18
   <211> 16
   <212> DNA
   <213> Artificial sequence
<220>
   <223> Single strand DNA oligonucleotide
<400> 18
   ggttgttgcg tttggg 16
<210> 19
   <211> 15
   <212> DNA
   <213> Artificial sequence
<220>
   <223> Single strand DNA oligonucleotide
<400> 19
   tgttggttgg ggagt 15
<210> 20
   <211> 15
   <212> DNA
   <213> Artificial sequence
<220>
   <223> Single strand DNA oligonucleotide
<400> 20
   tgttggtcgg ggagt 15
<210> 21
   <211> 15
   <212> DNA
   <213> Artificial sequence
<220>
   <223> Single strand DNA oligonucleotide
<400> 21
   ttttttttgg tgtga 15
<210> 22
   <211> 15
   <212> DNA
   <213> Artificial sequence
<220>
   <223> Single strand DNA oligonucleotide
<400> 22
   tttttttcgg tgtga 15
<210> 23
   <211> 15
   <212> DNA
   <213> Artificial sequence
<220>
   <223> Single strand DNA oligonucleotide
<400> 23
   agttttttgg tggtg 15
<210> 24
   <211> 15
   <212> DNA
   <213> Artificial sequence
<220>
   <223> Single strand DNA oligonucleotide
<400> 24
   agtttttcgg tggtg 15
<210> 25
   <211> 15
   <212> DNA
   <213> Artificial sequence
<220>
   <223> Single strand DNA oligonucleotide
<400> 25
   ggtgggttgg attag 15
<210> 26
   <211> 15
   <212> DNA
   <213> Artificial sequence
<220>
   <223> Single strand DNA oligonucleotide
<400> 26
   ggtgggtcgg attag 15
<210> 27
   <211> 15
   <212> DNA
   <213> Artificial sequence
<220>
   <223> Single strand DNA oligonucleotide
<400> 27
   tggggagtgg agggg 15
<210> 28
   <211> 15
   <212> DNA
   <213> Artificial sequence
<220>
   <223> Single strand DNA oligonucleotide
<400> 28
   gggggagcgg agggg 15
<210> 29
   <211> 15
   <212> DNA
   <213> Artificial sequence
<220>
   <223> Single strand DNA oligonucleotide
<400> 29
   tttttggcgt gagtg 15
<210> 30
   <211> 15
   <212> DNA
   <213> Artificial sequence
<220>
   <223> Single strand DNA oligonucleotide
<400> 30
   tttttggcgt gagtg 15
<210> 31
   <211> 15
   <212> DNA
   <213> Artificial sequence
<220>
   <223> Single strand DNA oligonucleotide
<400> 31
   gggggtgtgt ggggt 15
<210> 32
   <211> 15
   <212> DNA
   <213> Artificial sequence
<220>
   <223> Single strand DNA oligonucleotide
<400> 32
   gggggtgcgt ggggt 15
<210> 33
   <211> 15
   <212> DNA
   <213> Artificial sequence
<220>
   <223> Single strand DNA oligonucleotide
<400> 33
   gtgtaggtgg tgtta 15
<210> 34
   <211> 15.
   <212> DNA
   <213> Artificial sequence
<220>
   <223> Single strand DNA oligonucleotide
<400> 34
   gtgtaggcgg tgtta 15
<210> 35
   <211> 15
   <212> DNA
   <213> Artificial sequence
<220>
   <223> Single strand DNA oligonucleotide
<400> 35
   tttggtgtga gtggg 15
<210> 36
   <211> 15
   <212> DNA
   <213> Artificial sequence
<220>
   <223> Single strand DNA oligonucleotide
<400> 36
   tttggtgcga gtggg 15
<210> 37
   <211> 15
   <212> DNA
   <213> Artificial sequence
<220>
   <223> Single strand DNA oligonucleotide
<400> 37
   aatttggtgt tttta 15
<210> 38
   <211> 15
   <212> DNA
   <213> Artificial sequence
<220>
   <223> Single strand DNA oligonucleotide
<400> 38
   aatttggcgt tttta 15
<210> 39
   <211> 15
   <212> DNA
   <213> Artificial sequence
<220>
   <223> Single strand DNA oligonucleotide
<400> 39
   atatttgcgt tttgg 15
<210> 40
   <211> 15
   <212> DNA
   <213> Artificial sequence
<220>
   <223> Single strand DNA oligonucleotide
<400> 40
   atatttgcgt tttgg 15
<210> 41
   <211> 15
   <212> DNA
   <213> Artificial sequence
<220>
   <223> Single strand DNA oligonucleotide
<400> 41
   tgtgttttgg gttaa 15
<210> 42
   <211> 15
   <212> DNA
   <213> Artificial sequence
<220>
   <223> Single strand DNA oligonucleotide
<400> 42
   tgtgtttcgg gttaa 15
<210> 43
   <211> 15
   <212> DNA
   <213> Artificial sequence
<220>
   <223> Single strand DNA oligonucleotide
<400> 43
   ggtgtggtgt gtgga 15
<210> 44
   <211> 15
   <212> DNA
   <213> Artificial sequence
<220>
   <223> Single strand DNA oligonucleotide
<400> 44
   ggtgtggcgt gtgga 15
<210> 45
   <211> 15
   <212> DNA
   <213> Artificial sequence
<220>
   <223> Single strand DNA oligonucleotide
<400> 45
   tgtggcgtgt ggagt 15
<210> 46
   <211> 15
   <212> DNA
   <213> Artificial sequence
<220>
   <223> Single strand DNA oligonucleotide
<400> 46
   tgtggcgcgt ggagt 15
<210> 47
   <211> 15
   <212> DNA
   <213> Artificial sequence
<220>
   <223> Single strand DNA oligonucleotide
<400> 47
   tggagtttgg tgtgt 15
<210> 48
   <211> 15
   <212> DNA
   <213> Artificial sequence
<220>
   <223> Single strand DNA oligonucleotide
<400> 48
   tggagttcgg tgtgt 15
<210> 49
   <211> 15
   <212> DNA
   <213> Artificial sequence
<220>
   <223> Single strand DNA oligonucleotide
<400> 49
   agtttggtgt gtttt 15
<210> 50
   <211> 15
   <212> DNA
   <213> Artificial sequence
<220>
   <223> Single strand DNA oligonucleotide
<400> 50
   agtttggcgt gtttt 15
<210> 51
   <211> 15
   <212> DNA
   <213> Artificial sequence
<220>
   <223> Single strand DNA oligonucleotide
<400> 51
   aattttgcgt tagtt 15
<210> 52
   <211> 15
   <212> DNA
   <213> Artificial sequence
<220>
   <223> Single strand DNA oligonucleotide
<400> 52
   aattttgcgt tagtt 15
<210> 53
   <211> 15
   <212> DNA
   <213> Artificial sequence
<220>
   <223> Single strand DNA oligonucleotide
<400> 53
   gttagtttgg tggtt 15
<210> 54
   <211> 15
   <212> DNA
   <213> Artificial sequence
<220>
   <223> Single strand DNA oligonucleotide
<400> 54
   gttagttcgg tggtt 15
<210> 55
   <211> 24
   <212> DNA
   <213> Artificial sequence
<220>
   <223> Single strand DNA oligonucleotide
<400> 55
   tccagaatct gttccagagc gtgc 24
<210> 56
   <211> 24
   <212> DNA
   <213> Artificial sequence
<220>
   <223> Single strand DNA oligonucleotide
<400> 56
   gctgtgaagg ttgctgttcc tcat 24
<210> 57
   <211> 24
   <212> DNA
   <213> Artificial sequence
<220>
   <223> Single strand DNA oligonucleotide
<400> 57
   tagaatttgt tttagagtgt gtgt 24
<210> 58
   <211> 18
   <212> DNA
   <213> Artificial sequence
<220>
   <223> Single strand DNA oligonucleotide
<400> 58
   tttgttttag agcgtgcg 18
<210> 59
   <211> 20
   <212> DNA
   <213> Artificial sequence
<220>
   <223> Single strand DNA oligonucleotide
<400> 59
   aaaaccatcc tcaccctact 20
<210> 60
   <211> 29
   <212> DNA
   <213> Artificial sequence
<220>
   <223> Single strand DNA oligonucleotide
<400> 60
   agatttagtt aagtttaagg atggaagtg 29
<210> 61
   <211> 21
   <212> DNA
   <213> Artificial sequence
<220>
   <223> Single strand DNA oligonucleotide
<400> 61
   gggttgggaa gggtttattt t 21
<210> 62
   <211> 26
   <212> DNA
   <213> Artificial sequence
<220>
   <223> Single strand DNA oligonucleotide
<400> 62
   aaaaaccatc ctcaccctac tactac 26
<210> 63
   <211> 21
   <212> DNA
   <213> Artificial sequence
<220>
   <223> Single strand DNA oligonucleotide
<400> 63
   ggtttatttt tggttgttgt t 21
<210> 64
   <211> 21
   <212> DNA
   <213> Artificial sequence
<220>
   <223> Single strand DNA oligonucleotide
<400> 64
   tatttttggt tgttgtttaa g 21
<210> 65
   <211> 21
   <212> DNA
   <213> Artificial sequence
<220>
   <223> Single strand DNA oligonucleotide
<400> 65
   ttttggttgt tgttaagatt t 21
<210> 66
   <211> 21
   <212> DNA
   <213> Artificial sequence
<220>
   <223> Single strand DNA oligonucleotide
<400> 66
   ggtttatttt cggttgttgt t 21
<210> 67
   <211> 21
   <212> DNA
   <213> Artificial sequence
<220>
   <223> Single strand DNA oligonucleotide
<400> 67
   ggtttatttt tggtcgttgt t 21
<210> 68
   <211> 21
   <212> DNA
   <213> Artificial sequence
<220>
   <223> Single strand DNA oligonucleotide
<400> 68
   ggtttatttt tggttgtcgt t 21
<210> 69
   <211> 21
   <212> DNA
   <213> Artificial sequence
<220>
   <223> Single strand DNA oligonucleotide
<400> 69
   ggtttatttt cggtcgttgt t 21
<210> 70
   <211> 21
   <212> DNA
   <213> Artificial sequence
<220>
   <223> Single strand DNA oligonucleotide
<400> 70
   ggtttatttt tggtcgtcgt t 21
<210> 71
   <211> 21
   <212> DNA
   <213> Artificial sequence
<220>
   <223> Single strand DNA oligonucleotide
<400> 71
   ggtttatttt cggttgtcgt t 21
<210> 72
   <211> 21
   <212> DNA
   <213> Artificial sequence
<220>
   <223> Single strand DNA oligonucleotide
<400> 72
   ggtttatttt cggtcgtcgt t 21
<210> 73
   <211> 21
   <212> DNA
   <213> Artificial sequence
<220>
   <223> Single strand DNA oligonucleotide
<400> 73
   tattttcggt tgttgtttaa g 21
<210> 74
   <211> 21
   <212> DNA
   <213> Artificial sequence
<220>
   <223> Single strand DNA oligonucleotide
<400> 74
   tatttttggt cggtgtttaa g 21
<210> 75
   <211> 21
   <212> DNA
   <213> Artificial sequence
<220>
   <223> Single strand DNA oligonucleotide
<400> 75
   tatttttggt tgtcgtttaa g 21
<210> 76
   <211> 21
   <212> DNA
   <213> Artificial sequence
<220>
   <223> Single strand DNA oligonucleotide
<400> 76
   tattttcggt cgttgtttaa g 21
<210> 77
   <211> 21
   <212> DNA
   <213> Artificial sequence
<220>
   <223> Single strand DNA oligonucleotide
<400> 77
   tatttttggt cgtcgtttaa g 21
<210> 78
   <211> 20
   <212> DNA
   <213> Artificial sequence
<220>
   <223> Single strand DNA oligonucleotide
<400> 78
   tattttcggt gtcgtttaag 20
<210> 79
   <211> 21
   <212> DNA
   <213> Artificial sequence
<220>
   <223> Single strand DNA oligonucleotide
<400> 79
   tattttcggt cgtcgtttaa g 21
<210> 80
   <211> 21
   <212> DNA
   <213> Artificial sequence
<220>
   <223> Single strand DNA oligonucleotide
<400> 80
   tttcggttgt tgttaagatt t 21
<210> 81
   <211> 21
   <212> DNA
   <213> Artificial sequence
<220>
   <223> Single strand DNA oligonucleotide
<400> 81
   ttttggtcgt tgttaagatt t 21
<210> 82
   <211> 21
   <212> DNA
   <213> Artificial sequence
<220>
   <223> Single strand DNA oligonucleotide
<400> 82
   ttttggttgt cgttaagatt t 21
<210> 83
   <211> 21
   <212> DNA
   <213> Artificial sequence
<220>
   <223> Single strand DNA oligonucleotide
<400> 83
   tttcggtcgt tgttaagatt t 21
<210> 84
   <211> 21
   <212> DNA
   <213> Artificial sequence
<220>
   <223> Single strand DNA oligonucleotide
<400> 84
   ttttggtcgt cgttaagatt t 21
<210> 85
   <211> 21
   <212> DNA
   <213> Artificial sequence
<220>
   <223> Single strand DNA oligonucleotide
<400> 85
   tttcggttgt cgttaagatt t 21
<210> 86
   <211> 21
   <212> DNA
   <213> Artificial sequence
<220>
   <223> Single strand DNA oligonucleotide
<400> 86
   tttcggtcgt cgttaagatt t 21
<210> 87
   <211> 30
   <212> DNA
   <213> Artificial sequence
<220>
   <223> Single strand DNA oligonucleotide
<400> 87
   gttatatgga tttttttgtt aatttttttt 30
<210> 88
   <211> 21
   <212> DNA
   <213> Artificial sequence
<220>
   <223> Single strand DNA oligonucleotide
<400> 88
   taagatttat cgaggagttt t 21
<210> 89
   <211> 21
   <212> DNA
   <213> Artificial sequence
<220>
   <223> Single strand DNA oligonucleotide
<400> 89
   taagatttat tgaggagttt t 21
<210> 90
   <211> 21
   <212> DNA
   <213> Artificial sequence
<220>
   <223> Single strand DNA oligonucleotide
<400> 90
   tgttttagag tgtgtgtgaa g 21
<210> 91
   <211> 21
   <212> DNA
   <213> Artificial sequence
<220>
   <223> Single strand DNA oligonucleotide
<400> 91.
   tgttttagag cgtgtgtgaa g 21
<210> 92
   <211> 21
   <212 > DNA
   <213> Artificial sequence
<220>
   <223> Single strand DNA oligonucleotide
<400> 92
   tgttttagag tgtgcgtgaa g 21
<210> 93
   <211> 21
   <212> DNA
   <213> Artificial sequence
<220>
   <223> Single strand DNA oligonucleotide
<400> 93
   tgttttagag tgtgtgcgaa g 21
<210> 94
   <211> 21
   <212> DNA
   <213> Artificial sequence
<220>
   <223> Single strand DNA oligonucleotide
<400> 94
   tgttttagag cgtgcgtgaa g 21
<210> 95
   <211> 21
   <212> DNA
   <213> Artificial sequence
<220>
   <223> Single strand DNA oligonucleotide
<400> 95
   tgttttagag cgtgtgcgaa g 21
<210> 96
   <211> 21
   <212> DNA
   <213> Artificial sequence
<220>
   <223> Single strand DNA oligonucleotide
<400> 96
   tgttttagag tgtgcgcgaa g 21
<210> 97
   <211> 21
   <212> DNA
   <213> Artificial sequence
<220>
   <223> Single strand DNA oligonucleotide
<400> 97
   tgttttagag cgtgcgcgaa g 21
<210> 98
   <211> 21
   <212> DNA
   <213> Artificial sequence
<220>
   <223> Single strand DNA oligonucleotide
<400> 98
   ttagagtgtg tgtgaagtga t 21
<210> 99
   <211> 21
   <212> DNA
   <213> Artificial sequence
<220>
   <223> Single strand DNA oligonucleotide
<400> 99
   ttagagcgtg tgtgaagtga t 21

<210> 100
   <211> 21
   <212> DNA
   <213> Artificial sequence
<220>
   <223> Single strand DNA oligonucleotide
<400> 100
   ttagagtgtg cgtgaagtga t 21
<210> 101
   <211> 21
   <212> DNA
   <213.> Artificial sequence
<220>
   <223> Single strand DNA oligonucleotide
<400> 101
   ttagagtgtg tgcgaagtga t 21
<210> 102
   <211> 21
   <212> DNA
   <213> Artificial sequence
<220>
   <223> Single strand DNA oligonucleotide
<400> 102
   ttagagcgtg cgtgaagtga t 21
<210> 103
   <211> 21
   <212> DNA
   <213> Artificial sequence
<220>
   <223> Single strand DNA oligonucleotide
<400> 103
   ttagagcgtg tgcgaagtga t 21
<210> 104
   <211> 21
   <212> DNA
   <213> Artificial sequence
<220>
   <223> Single strand DNA oligonucleotide
<400> 104
   ttagagtgtg cgcgaagtga t 21
<210> 105
   <211> 21
   <212> DNA
   <213> Artificial sequence
<220>
   <223> Single strand DNA oligonucleotide
<400> 105.
   ttagagcgtg cgcgaagtga t 21
<210> 106
   <211> 21
   <212> DNA
   <213> Artificial sequence
<220>
   <223> Single strand DNA oligonucleotide
<400> 106
   agagtgtgtg tgaagtgatt t 21
<210> 107
   <211> 21
   <212> DNA
   <213> Artificial sequence
<220>
   <223> Single strand DNA oligonucleotide
<400> 107
   agagcgtgtg tgaagtgatt t 21
<210> 108
   <211> 21
   <212> DNA
   <213> Artificial sequence
<220>
   <223> Single strand DNA oligonucleotide
<400> 108
   agagtgtgcg tgaagtgatt t 21
<210> 109
   <211> 21
   <212> DNA
   <213> Artificial sequence
<220>
   <223> Single strand DNA oligonucleotide
<400> 109
   agagtgtgtg cgaagtgatt t 21
<210> 110
   <211> 21
   <212> DNA
   <213> Artificial sequence
<220>
   <223> Single strand DNA oligonucleotide
<400> 110
   agagcgtgcg tgaagtgatt t 21
<210> 111
   <211> 21
   <212> DNA
   <213> Artificial sequence
<220>
   <223> Single strand DNA oligonucleotide
<400> 111
   agagcgtgtg cgaagtgatt t 21
<210> 112
   <211> 21
   <212> DNA
   <213> Artificial sequence
<220>
   <223> Single strand DNA oligonucleotide
<400> 112
   agagtgtgcg cgaagtgatt t 21
<210> 113
   <211> 22
   <212> DNA
   <213> Artificial sequence
<220>
   <223> Single strand DNA oligonucleotide
<400> 113
   agagcgtgcg cgcaagtgat tt 22
<210> 114
   <211> 21
   <212> DNA
   <213> Artificial sequence
<220>
   <223> Single strand DNA oligonucleotide
<400> 114
   atttagaatt tgggttttag g 21
<210> 115
   <211> 21
   <212> DNA
   <213> Artificial sequence
<220>
   <223> Single strand DNA oligonucleotide
<400> 115
   atttagaatt cgggttttag g 21
<210> 116
   <211> 21
   <212> DNA
   <213> Artificial sequence
<220>
   <223> Single strand DNA oligonucleotide
<400> 116
   atttagaggt tgtgagtgta g 21
<210> 117
   <211> 21
   <212> DNA
   <213> Artificial sequence
<220>
   <223> Single strand DNA oligonucleotide
<400> 117
   atttagaggt cgcgagcgta g 21
<210> 118
   <211> 21
   <212> DNA
   <213> Artificial sequence
<220>
   <223> Single strand DNA oligonucleotide
<400> 118
   atttagaggt cgtgagtgta g 21
<210> 119
   <211> 21
   <212> DNA
   <213> Artificial sequence
<220>
   <223> Single strand DNA oligonucleotide
<400> 119
   atttagaggt tgcgagtgta g 21
<210> 120
   <211> 21
   <212> DNA
   <213> Artificial sequence
<220>
   <223> Single strand DNA oligonucleotide
<400> 120
   atttagaggt tgtgagcgta g 21
<210> 121
   <211> 21
   <212> DNA
   <213> Artificial sequence
<220>
   <223> Single strand DNA oligonucleotide
<400> 121
   atttagaggt cgcgagtgta g 21
<210> 122
   <211> 21
   <212> DNA
   <213> Artificial sequence
<220>
   <223> Single strand DNA oligonucleotide
<400> 122
   atttagaggt cgtgagcgta g 21
<210> 123
   <211> 21
   <212> DNA
   <213> Artificial sequence
<220>
   <223> Single strand DNA oligonucleotide
<400> 123
   atttagaggt tgcgagcgta g 21
<210> 124
   <211> 21
   <212> DNA
   <213> Artificial sequence
<220>
   <223> Single strand DNA oligonucleotide
<400>. 124
   atttagaggt cgcgagcgta g 21
<210> 125
   <211> 21
   <212> DNA
   <213> Artificial sequence
<220>
   <223> Single strand DNA oligonucleotide
<400> 125
   atttagaggt tgtgagtgta g 21
<210> 126
   <211> 21
   <212> DNA
   <213> Artificial sequence
<220>
   <223> Single strand DNA oligonucleotide
<400> 126
   ttagaggtcg cgagcgtagt a 21
<210> 127
   <211> 21
   <212> DNA
   <213> Artificial sequence
<220>
   <223> Single strand DNA oligonucleotide
<400> 127
   ttagaggtcg tgagtgtagt a 21
<210> 128
   <211> 21
   <212> DNA
   <213> Artificial sequence
<220>
   <223> Single strand DNA oligonucleotide
<400> 128
   ttagaggttg cgagtgtagt a 21
<210> 129
   <211> 21
   <212> DNA
   <213> Artificial sequence
<220>
   <223> Single strand DNA oligonucleotide
<400> 129
   ttagaggttg tgagcgtagt a 21
<210> 130
   <211> 21
   <212> DNA
   <213> Artificial sequence
<220>
   <223> Single strand DNA oligonucleotide
<400> 130
   ttagaggtcg cgagtgtagt a 21
<210> 131
   <211> 21
   <212> DNA
   <213> Artificial sequence
<220>
   <223> Single strand DNA oligonucleotide
<400> 131
   ttagaggtcg tgagcgtagt a 21
<210> 132
   <211> 21
   <212> DNA
   <213> Artificial sequence
<220>
   <223> Single strand DNA oligonucleotide
<400> 132
   ttagaggttg cgagcgtagt a 21
<210> 133
   <211> 21
   <212> DNA
   <213> Artificial sequence
<220>
   <223> Single strand DNA oligonucleotide
<400> 133
   ttagaggtcg cgagcgtagt a 21
<210> 134
   <211> 21
   <212> DNA
   <213> Artificial sequence
<220>
   <223> Single strand DNA oligonucleotide
<400> 134
   aggttgtgag tgtagtattt t 21
<210> 135
   <211> 21
   <212> DNA
   <213> Artificial sequence
<220>
   <223> Single strand DNA oligonucleotide
<400> 135
   aggtcgcgag cgtagtattt t 21
<210> 136
   <2111> 21
   <212> DNA
   <213> Artificial sequence
<220>
   <223> Single strand DNA oligonucleotide
<400> 136
   aggtcgtgag tgtagtattt t 21
<210> 137
   <211> 21
   <212> DNA
   <213> Artificial sequence
<220>
   <223> Single strand DNA oligonucleotide
<400> 137
   aggttgcgag tgtagtattt t 21
<210> 138
   <211> 21
   <212> DNA
   <213> Artificial sequence
<220>
   <223> Single strand DNA oligonucleotide
<400> 138
   aggttgtgag cgtagtattt t 21
<210> 139
   <211> 21
   <212> DNA
   <213> Artificial sequence
<220>
   <223> Single strand DNA oligonucleotide
<400> 139
   aggtcgcgag tgtagtattt t 21
<210> 140
   <211> 21
   <212> DNA
   <213> Artificial sequence
<220>
   <223> Single strand DNA oligonucleotide
<400> 140
   aggtcgtgag cgtagtattt t 21
<210> 141
   <211> 21
   <212> DNA
   <213> Artificial sequence
<220>
   <223> Single strand DNA oligonucleotide
<400> 141
   aggttgcgag cgtagtattt t 21
<210> 142
   <211> 21
   <212> DNA
   <213> Artificial sequence
<220>
   <223> Single strand DNA oligonucleotide
<400> 142
   aggtcgcgag cgtagtattt t 21
<210> 143
   <211> 21
   <212> DNA
   <213> Artificial sequence
<220>
   <223> Single strand DNA oligonucleotide
<400> 143
   tagtattttt tggtgttagt t 21
<210> 144
   <211> 21
   <212> DNA
   <213> Artificial sequence
<220>
   <223> Single strand DNA oligonucleotide
<400> 144
   tagtattttt tggcgttagt t 21
<210> 145
   <211> 21
   <212> DNA
   <213> Artificial sequence
<220>
   <223> Single strand DNA oligonucleotide
<400> 145
   tagtattttt cggtgttagt t 21
<210> 146
   <211> 21
   <212> DNA
   <213> Artificial sequence
<220>
   <223> Single strand DNA oligonucleotide
<400> 146
   tagtattttt cggcgttagt t 21
<210> 147
   <211> 24
   <212> DNA
   <213> Artificial sequence
<220>
   <223> Single strand DNA oligonucleotide
<400> 147
   tagtattttt tggtgttagt ttgt 24
<210> 148
   <211> 24
   <212> DNA
   <213> Artificial sequence
<220>
   <223> Single strand DNA oligonucleotide
<400> 148
   tagtattttt tggcgttagt ttgt 24
<210> 149
   <211> 24
   <212> DNA
   <213> Artificial sequence
<220>
   <223> Single strand DNA oligonucleotide
<400> 149
   tagtattttt cggtgttagt ttgt 24
<210> 150
   <211> 24
   <212> DNA
   <213> Artificial sequence
<220>
   <223>Single strand DNA oligonucleotide
<400> 150
   tagtattttt cggcgttagt ttgt 24
<210> 151
   <211> 29
   <212> DNA
   <213> Artificial sequence
<220>
   <223> Single strand DNA oligonucleotide
<400> 151
   tctctactac tactttaaaa ctacaaaac 29
<210> 152
   <211> 30
   <212> DNA
   <213> Artificial sequence
<220>
   <223> Single strand DNA oligonucleotide
<400> 152
   ggttttagtt atatggattt ttttgttaat 30
<210> 153
   <211> 21
   <212> DNA
   <213> Artificial sequence
<220>
   <223> Single strand DNA oligonucleotide
<400> 153
   tttttgtttg cgagttgggt g 21
<210> 154
   <211> 20
   <212> DNA
   <213> Artificial sequence
<220>
   <223> Single strand DNA oligonucleotide
<400> 154
   ttttgtttgt gagtcgggtg 20
<210> 155
   <211> 20
   <212> DNA
   <213> Artificial sequence
<220>
   <223> Single strand DNA oligonucleotide
<400> 155
   ttttgtttgt gagttgggcg 20
<210> 156
   <211> 20
   <212> DNA
   <213> Artificial sequence
<220>
   <223> Single strand DNA oligonucleotide
<400> 156
   ttttgtttgc gagtcgggtg 20
<210> 157
   <211> 20
   <212> DNA
   <213> Artificial sequence
<220>
   <223> Single strand DNA oligonucleotide
<400> 157
   ttttgtttgc gagttgggcg 20
<210> 158
   <211> 20
   <212> DNA
   <213> Artificial sequence
<220>
   <223> Single strand DNA oligonucleotide
<400> 158
   ttttgtttgt gagtcgggcg 20
<210> 159
   <211> 20
   <212> DNA
   <213> Artificial sequence
<220>
   <223> Single strand DNA oligonucleotide
<400> 159
   ttttgtttgc gagtcgggcg 20
<210> 160
   <211> 21
   <212> DNA
   <213> Artificial sequence
<220>
   <223> Single strand DNA oligonucleotide
<400> 160
   gtttgtgagt tgggtgagtg a 21
<210> 161
   <211> 21
   <212> DNA
   <213> Artificial sequence
<220>
   <223> Single strand DNA oligonucleotide
<400> 161
   gtttgcgagt tgggtgagtg a 21
<210> 162
   <211> 21
   <212> DNA
   <213> Artificial sequence
<220>
   <223> Single strand DNA oligonucleotide
<400> 162
   gtttgtgagt cgggtgagtg a 21
<210> 163
   <211> 21
   <212> DNA
   <213> Artificial sequence
<220>
   <223> Single strand DNA oligonucleotide
<400> 163
   gtttgtgagt tgggcgagtg a 21
<210> 164
   <211> 21
   <212> DNA
   <213> Artificial sequence
<220>
   <223> Single strand DNA oligonucleotide
<400> 164
   gtttgcgagt cgggtgagtg a 21
<210> 165
   <211> 21
   <212> DNA
   <213> Artificial sequence
<220>
   <223> Single strand DNA oligonucleotide
<400> 165
   gtttgcgagt tgggcgagtg a 21
<210> 166
   <211> 21
   <212> DNA
   <213> Artificial sequence
<220>
   <223> Single strand DNA oligonucleotide
<400> 166
   gtttgtgagt cgggcgagtg a 21
<210> 167
   <211> 21
   <212> DNA
   <213> Artificial sequence
<220>
   <223> Single strand DNA oligonucleotide
<400> 167
   gtttgcgagt cgggcgagtg a 21
<210> 168
   <211> 22
   <212> DNA
   <213> Artificial sequence
<220>
   <223> Single strand DNA oligonucleotide
<400> 168
   gtgagttggg tgagtgaagt tg 22
<210> 169
   <211> 22
   <212> DNA
   <213> Artificial sequence
<220>
   <223> Single strand DNA oligonucleotide
<400> 169
   gcgagttggg tgagtgaagt tg 22
<210> 170
   <211> 22
   <212> DNA
   <213> Artificial sequence
<220>
   <223> Single strand DNA oligonucleotide
<400> 170
   gtgagtcggg tgagtgaagt tg 22
<210> 171
   <211> 22
   <212> DNA
   <213> Artificial sequence
<220>
   <223> Single strand DNA oligonucleotide
<400> 171
   gtgagttggg cgagtgaagt tg 22
<210> 172
   <211> 22
   <212> DNA
   <213> Artificial sequence
<220>
   <223> Single strand DNA oligonucleotide
<400> 172
   gtgagttggg tgagtgaagt cg 22
<210> 173
   <211> 22
   <212> DNA
   <213> Artificial sequence
<220>
   <223> Single strand DNA oligonucleotide
<400> 173
   gtgagttggg cgagtgaagt cg 22
<210> 174
   <211> 22
   <212> DNA
   <213> Artificial sequence
<220>
   <223> Single strand DNA oligonucleotide
<400> 174
   gtgagtcggg tgagtgaagt cg 22
<210> 175
   <211> 22
   <212> DNA
   <213> Artificial sequence
<220>
   <223> Single strand DNA oligonucleotide
<400> 175
   gcgagttggg tgagtgaagt cg 22
<210> 176
   <211> 22
   <212> DNA
   <213> Artificial sequence
<220>
   <223> Single strand DNA oligonucleotide
<400> 176
   gtgagtcggg cgagtgaagt tg 22
<210> 177
   <211> 22
   <212> DNA
   <213> Artificial sequence
<220>
   <223> Single strand DNA oligonucleotide
<400> 177
   gcgagttggg cgagtgaagt tg 22
<210> 178
   <211> 22
   <212> DNA
   <213> Artificial sequence
<220>
   <223> Single strand DNA oligonucleotide
<400> 178
   gcgagtcggg cgagtgaagt cg 22
<210> 179
   <211> 22
   <212> DNA
   <213> Artificial sequence
<220>
   <223> Single strand DNA oligonucleotide
<400> 179
   gcgagtcggg tgagtgaagt tg 22
<210> 180
   <211> 22
   <212> DNA
   <213> Artificial sequence
<220>
   <223> Single strand DNA oligonucleotide
<400> 180
   gcgagtcggg cgagtgaagt tg 22
<210> 181
   <211> 22
   <212> DNA
   <213> Artificial sequence
<220>
   <223> Single strand DNA oligonucleotide
<400> 181
   gcgagtcggg tgagtgaagt cg 22
<210> 182
   <211> 22
   <212> DNA
   <213> Artificial sequence
<220>
   <223> Single strand DNA oligonucleotide
<400> 182
   gcgagttggg cgagtgaagt cg 22
<210> 183
   <211> 22
   <212> DNA
   <213> Artificial sequence
<220>
   <223> Single strand DNA oligonucleotide
<400> 183
   gcgagtcggg cgagtgaagt cg 22
<210> 184
   <211> 21
   <212> DNA
   <213> Artificial sequence
<220>
   <223> Single strand DNA oligonucleotide
<400> 184
   tgagtgaagt tgagtgtgga g 21
<210> 185
   <211> 22
   <212> DNA
   <213> Artificial sequence
<220>
   <223> Single strand DNA oligonucleotide
<400> 185
   cgagtgaagt tgagtgctgg ag 22
<210> 186
   <211> 21
   <212> DNA
   <213> Artificial sequence
<220>
   <223> Single strand DNA oligonucleotide
<400> 186
   tgagtgaagt cgagtgtgga g 21
<210> 187
   <211> 21
   <212> DNA
   <213> Artificial sequence
<220>
   <223> Single strand DNA oligonucleotide
<400> 187
   tgagtgaagt tgagcgtgga g 21
<210> 188
   <211> 21
   <212> DNA
   <213> Artificial sequence
<220>
   <223> Single strand DNA oligonucleotide
<400> 188
   tgagtgaagt tgagtgcgga g 21
<210> 189
   <211> 21
   <212> DNA
   <213> Artificial sequence
<220>
   <223> Single strand DNA oligonucleotide
<400> 189
   cgagtgaagt cgagtgtgga g 21
<210> 190
   <211> 21
   <212> DNA
   <213> Artificial sequence
<220>
   <223> Single strand DNA oligonucleotide
<400> 190
   tgagtgaagt tgagcgcgga g 21
<210> 191
   <211> 21.
   <212> DNA
   <213> Artificial sequence
<220>
   <223> Single strand DNA oligonucleotide
<400> 191
   tgagtgaagt cgagtgcgga g 21
<210> 192
   <211> 21
   <212> DNA
   <213> Artificial sequence
<220>
   <223> Single strand DNA oligonucleotide
<400> 192
   cgagtgaagt tgagcgtgga g 21
<210> 193
   <211> 21
   <212> DNA
   <213> Artificial sequence
<220>
   <223> Single strand DNA oligonucleotide
<400> 193
   tgagtgaagt cgagcgtgga g 21
<210> 194
   <211> 21
   <212> DNA
   <213> Artificial sequence
<220>
   <223> Single strand DNA oligonucleotide
<400> 194
   cgagtgaagt tgagcgtgga g 21
<210> 195
   <211> 21
   <212> DNA
   <213> Artificial sequence
<220>
   <223> Single strand DNA oligonucleotide
<400> 195
   cgagtgaagt cgagcgtgga g. 21
<210> 196
   <211> 21
   <212> DNA
   <213> Artificial sequence
<220>
   <223> Single strand DNA oligonucleotide
<400> 196
   tgagtgaagt cgagcgcgga g 21
<210> 197
   <211> 21
   <212> DNA
   <213> Artificial sequence
<220>
   <223> Single strand DNA oligonucleotide
<400> 197
   cgagtgaagt tgagcgcgga g 21
<210> 198
   <211> 21
   <212> DNA
   <213> Artificial sequence
<220>
   <223> Single strand DNA oligonucleotide
<400> 198
   cgagtgaagt cgagtgcgga g 21
<210> 199
   <211> 21
   <212> DNA
   <213> Artificial sequence
<220>
   <223> Single strand DNA oligonucleotide
<400> 199
   cgagtgaagt cgagcgcgga g 21
<210> 200
   <211> 21
   <212> DNA
   <213> Artificial sequence
<220>
   <223> Single strand DNA oligonucleotide
<400> 200
   tgaagttgag tgtggaggtg a 21
<210> 201
   <211> 22
   <212> DNA
   <213> Artificial sequence
<220>
   <223> Single strand DNA oligonucleotide
<400> 201
   tgaagtcgag tgctggaggt ga 22
<210> 202
   <211> 21
   <212> DNA
   <213> Artificial sequence
<220>
   <223> Single strand DNA oligonucleotide
<400> 202
   tgaagttgag cgtggaggtg a 21
<210> 203
   <211> 21
   <212> DNA
   <213> Artificial sequence
<220>
   <223> Single strand DNA oligonucleotide
<400> 203
   tgaagttgag tgtggaggcg a 21
<210> 204
   <211> 21
   <212> DNA
   <213> Artificial sequence
<220>
   <223> Single strand DNA oligonucleotide
<400> 204
   tgaagttgag tgcggaggcg a 21
<210> 205
   <211> 21
   <212> DNA
   <213> Artificial sequence
<220>
   <223> Single strand DNA oligonucleotide
<400> 205
   tgaagttgag cgtggaggcg a 21
<210> 206
   <211> 21
   <212> DNA
   <213> Artificial sequence
<220>
   <223> Single strand DNA oligonucleotide
<400> 206
   tgaagtcgag tgtggaggcg a 21
<210> 207
   <211> 21
   <212> DNA
   <213> Artificial sequence
<220>
   <223> Single strand DNA oligonucleotide
<400> 207
   tgaagttgag cgcggaggtg a 21
<210> 208
   <211> 21
   <212> DNA
   <213> Artificial sequence
<220>
   <223> Single strand DNA oligonucleotide
<400> 208
   tgaagtcgag tgcggaggtg a 21
<210> 209
   <211> 21
   <212> DNA
   <213> Artificial sequence
<220>
   <223> Single strand DNA oligonucleotide
<400> 209
   tgaagtcgag cgtggaggtg a 21
<210> 210
   <211> 21
   <212> DNA
   <213> Artificial sequence
<220>
   <223> Single strand DNA oligonucleotide
<400> 210
   tgaagtcgag cgcggaggtg a 21
<210> 211
   <211> 21
   <212> DNA
   <213> Artificial sequence
<220>
   <223> Single strand DNA oligonucleotide
<400> 211
   tgaagtcgag cgtggaggcg a 21
<210> 212
   <211> 21
   <212> DNA
   <213> Artificial sequence
<220>
   <223> Single strand DNA oligonucleotide
<400> 212
   tgaagtcgag tgcggaggcg a 21
<210> 213
   <211> 21
   <212> DNA
   <213> Artificial sequence
<220>
   <223> Single strand DNA oligonucleotide
<400> 213
   tgaagttgag cgcggaggcg a 21
<210> 214
   <211> 21
   <212> DNA
   <213> Artificial sequence
<220>
   <223> Single strand DNA oligonucleotide
<400> 214
   tgaagtcgag cgcggaggcg a 21
<210> 215
   <211> 21
   <212> DNA
   <213> Artificial sequence
<220>
   <223> Single strand DNA oligonucleotide
<400> 215
   aagttgagtg tggaggtgag t 21
<210> 216
   <211> 22
   <212> DNA
   <213> Artificial sequence
<220>
   <223> Single strand DNA oligonucleotide
<400> 216
   aagtcgagtg ctggaggtga gt 22
<210> 217
   <211> 21
   <212> DNA
   <213> Artificial sequence
<220>
   <223> Single strand DNA oligonucleotide
<400> 217
   aagttgagcg tggaggtgag t 21
<210> 218
   <211> 21
   <212> DNA
   <213> Artificial sequence
<220>
   <223> Single strand DNA oligonucleotide
<400> 218
   aagttgagtg tggaggcgag t 21
<210> 219
   <211> 21
   <212> DNA
   <213> Artificial sequence
<220>
   <223> Single strand DNA oligonucleotide
<400> 219
   aagttgagtg cggaggcgag t 21
<210> 220
   <211> 21
   <212> DNA
   <213> Artificial sequence
<220>
   <223> Single strand DNA oligonucleotide
<400> 220
   aagttgagcg tggaggcgag t 21
<210> 221
   <211> 21
   <212> DNA
   <213> Artificial sequence
<220>
   <223> Single strand DNA oligonucleotide
<400> 221
   aagtcgagtg tggaggcgag t 21
<210> 222
   <211> 21
   <212> DNA
   <213> Artificial sequence
<220>
   <223> Single strand DNA oligonucleotide
<400> 222
   aagttgagcg cggaggtgag t 21
<210> 223
   <211> 21
   <212> DNA
   <213> Artificial sequence
<220>
   <223> Single strand DNA oligonucleotide
<400> 223
   aagtcgagtg cggaggtgag t 21
<210> 224
   <211> 21
   <212> DNA
   <213> Artificial sequence
<220>
   <223> Single strand DNA oligonucleotide
<400> 224
   aagtcgagcg tggaggtgag t 21
<210> 225
   <211> 21
   <212> DNA
   <213> Artificial sequence
<220>
   <223> Single strand DNA oligonucleotide
<400> 225
   aagtcgagcg cggaggtgag t 21
<210> 226
   <211> 21
   <212> DNA
   <213> Artificial sequence
<220>
   <223> Single strand DNA oligonucleotide
<400> 226
   aagtcgagcg tggaggcgag t 21
<210> 227
   <211> 21
   <212> DNA
   <213> Artificial sequence
<220>
   <223> Single strand DNA oligonucleotide
<400> 227
   aagtcgagtg cggaggcgag t 21
<210> 228
   <211> 21
   <212> DNA
   <213> Artificial sequence
<220>
   <223> Single strand DNA oligonucleotide
<400> 228
   aagttgagcg cggaggcgag t 21
<210> 229
   <211> 21
   <212> DNA
   <213> Artificial sequence
<220>
   <223> Single strand DNA oligonucleotide
<400> 229
   aagtcgagcg cggaggcgag t 21
<210> 230
   <211> 21
   <212> DNA
   <213> Artificial sequence
<220>
   <223> Single strand DNA oligonucleotide
<400> 230
   agtgtggagg tgagtaggga t 21
<210> 231
   <211> 20
   <212> DNA
   <213> Artificial sequence
<220>
   <223> Single strand DNA oligonucleotide
<400> 231
   gtgcggaggt gagtagggat 20
<210> 232
   <211> 20
   <212> DNA
   <213> Artificial sequence
<220>
   <223> Single strand DNA oligonucleotide
<400> 232
   gcgtggaggt gagtagggat 20
<210> 233
   <211> 20
   <212> DNA
   <213> Artificial sequence
<220>
   <223> Single strand DNA oligonucleotide
<400> 233
   gtgtggaggc gagtagggat 20
<210> 234
   <211> 20
   <212> DNA
   <213> Artificial sequence
<220>
   <223> Single strand DNA oligonucleotide
<400> 234
   gtgcggaggc gagtagggat 20
<210> 235
   <211> 20
   <212> DNA
   <213> Artificial sequence
<220>
   <223> Single strand DNA oligonucleotide
<400> 235
   gcgtggaggc gagtagggat 20
<210> 236
   <211> 20
   <212> DNA
   <213> Artificial sequence
<220>
   <223> Single strand DNA oligonucleotide
<400> 236
   gcgcggaggt gagtagggat 20
<210> 237
   <211> 20
   <212> DNA.
   <213> Artificial sequence
<220>
   <223> Single strand DNA oligonucleotide
<400> 237
   gcgcggaggc gagtagggat 20
<210> 238
   <211> 21
   <212> DNA
   <213> Artificial sequence
<220>
   <223> Single strand DNA oligonucleotide
<400> 238
   tgtttttggt tgttggggtg t 21
<210> 239
   <211> 21
   <212> DNA
   <213> Artificial sequence
<220>
   <223> Single strand DNA oligonucleotide
<400> 239
   tgtttttggt cgttggggtg t 21
<210> 240
   <211> 21
   <212> DNA
   <213> Artificial sequence
<220>
   <223> Single strand DNA oligonucleotide
<400> 240
   tgtttttggt tgttggggcg t 21
<210> 241
   <211> 21
   <212> DNA
   <213> Artificial sequence
<220>
   <223> Single strand DNA oligonucleotide
<400> 241
   tgtttttggt cgttggggcg t 21
<210> 242
   <211> 21
   <212> DNA
   <213> Artificial sequence
<220>
   <223> Single strand DNA oligonucleotide
<400> 242
   gttgttgggg tgttttgtag t 21
<210> 243
   <211> 21
   <212> DNA
   <213> Artificial-sequence
<220>
   <223> Single strand DNA oligonucleotide
<400> 243
   gtcgttgggg tgttttgtag t 21
<210> 244
   <211> 21
   <212> DNA
   <213> Artificial sequence
<220>
   <223> Single strand DNA oligonucleotide
<400> 244
   gttgttgggg cgttttgtag t 21
<210> 245
   <211> 21
   <212> DNA
   <213> Artificial sequence
<220>
   <223> Single strand DNA oligonucleotide
<400> 245
   gtcgttgggg cgttttgtag t 21
<210> 246
   <211> 21
   <212> DNA
   <213> Artificial sequence
<220>
   <223> Single strand DNA oligonucleotide
<400> 246
   tttttgtttg tgagtcgggt g 21
<210> 247
   <211> 25
   <212> DNA
   <213> Artificial sequence
<220>
   <223> Single strand DNA oligonucleotide
<400> 247
   ttttagtttt atttggtttt taggt 25
<210> 248
   <211> 25
   <212> DNA
   <213> Artificial sequence
<220>
   <223> Single strand DNA oligonucleotide
<400> 248
   aaaaaacctt aaccttcaca aaatc 25
<210> 249
   <211> 25
   <212> DNA
   <213> Artificial sequence
<220>
   <223> Single strand DNA oligonucleotide
<400> 249
   attgtttaag attttagggt tagta 25